# EUROPEAN PATENT APPLICATION

(11) **EP 1 574 501 A1**
(43) Date of publication of application: **14.09.2005**
(21) Application number: 04290667.7
(22) Date of filing: 11.03.2004
(51) Int. Cl.: C07D 215/26, C07D 401/12, A61K 31/4704, A61K 31/4725, A61P 11/08, A61P 11/06

(54) **Quinolinone derivatives, pharmaceutical compositions containing them and their use**

(71) Applicant: Pfizer Limited, Kent CT13 9NJ (GB)
(72) Inventor: The designation of the inventor has not yet been filed
(74) Representative: Laurent, Claire

(57) **Abstract**

The invention relates to compounds of formula (1) 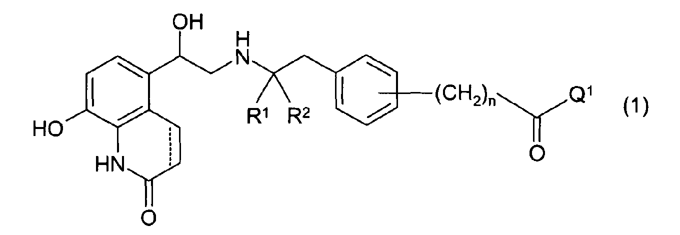 and to processes for the preparation of, intermediates used in the preparation of, compositions containing and the uses of, such derivatives. The compounds according to the present invention are useful in numerous diseases, disorders and conditions, in particular inflammatory, allergic and respiratory diseases, disorders and conditions.

## Description

This invention relates to β2 agonists of general formula: 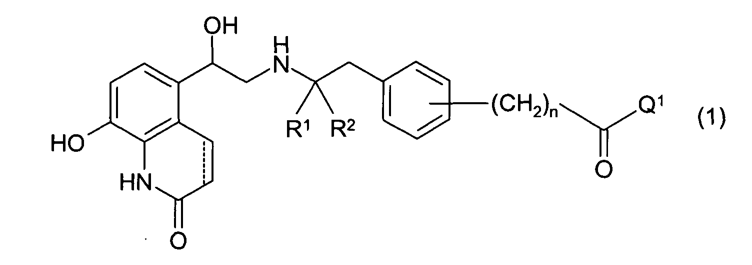 in which R¹, R², n and Q¹ have the meanings indicated below, and to processes for the preparation of, compositions containing and the uses of such derivatives.

Adrenoceptors are members of the large G-protein coupled receptor super-family. The adrenoceptor subfamily is itself divided into the α and β subfamilies with the β sub-family being composed of at least 3 receptor subtypes: β1, β2 and β3. These receptors exhibit differential expression patterns in tissues of various systems and organs of mammals. β2 adrenergic (β2) receptors are mainly expressed in smooth muscle cells (e.g. vascular, bronchial, uterine or intestinal smooth muscles), whereas β3 adrenergic receptors are mainly expressed in fat tissues (therefore β3 agonists could potentially be useful in the treatment of obesity and diabetes) and β1 adrenergic receptors are mainly expressed in cardiac tissues (therefore β1 agonists are mainly used as cardiac stimulants).

The pathophysiology and treatments of airway diseases have been extensively reviewed in the literature (for reference see Barnes, P.J. Chest, 1997, 111:2, pp 17S-26S and Bryan, S.A. et al, Expert Opinion on investigational drugs, 2000, 9:1, pp25-42) and therefore only a brief summary will be included here to provide some background information.

Glucocorticosteroids, anti-leukotrienes, theophylline, cromones, anticholinergics and β2 agonists constitute drug classes that are currently used to treat allergic and non-allergic airways diseases such as asthma and chronic obstructive airways disease (COPD). Treatment guidelines for these diseases include both short and long acting inhaled β2 agonists. Short acting, rapid onset β2 agonists are used for "rescue" bronchodilation, whereas, long-acting forms provide sustained relief and are used as maintenance therapy.

Bronchodilation is mediated via agonism of the β2 adrenoceptor expressed on airway smooth muscle cells, which results in relaxation and hence bronchodilation. Thus, as functional antagonists, β2 agonists can prevent and reverse the effects of all bronchoconstrictor substances, including leukotriene D4 (LTD4), acetylcholine, bradykinin, prostaglandins, histamine and endothelins. Because β2 receptors are so widely distributed in the airway, β2 agonists may also affect other types of cells that play a role in asthma. For example, it has been reported that β2 agonists may stabilize mast cells. The inhibition of the release of bronchoconstrictor substances may be how β2 agonists block the bronchoconstriction induced by allergens, exercise and cold air. Furthermore, β2 agonists inhibit cholinergic neurotransmission in the human airway, which can result in reduced cholinergic-reflex bronchoconstriction.

In addition to the airways, it has also been established that β2 adrenoceptors are also expressed in other organs and tissues and thus β2 agonists, such as those described in the present invention, may have application in the treatment of other diseases such as, but not limited to those of the nervous system, premature labor, congestive heart failure, depression, inflammatory and allergic skin diseases, psoriasis, proliferative skin diseases, glaucoma and in conditions where there is an advantage in lowering gastric acidity, particularly in gastric and peptic ulceration.

However, numerous β2 agonists are limited in their use due to their low selectivity or adverse side-effects driven by high systemic exposure and mainly mediated through action at β2 adrenoreceptors expressed outside the airways (muscle tremor, tachycardia, palpitations, restlessness). Therefore there is a need for improved agents in this class.

Accordingly, there is still a need for novel β₂ agonists that would have an appropriate pharmacological profile, for example in terms of potency, selectivity, pharmacokinetics or duration of action. In this context, the present invention relates to novel β2 agonists.

Various quinolinone derivatives have already been disclosed. For example, WO00/75114 discloses compounds active as β2 agonist, of formula:

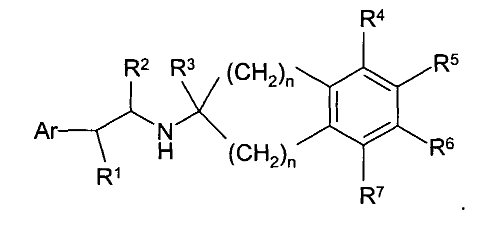

EP147719 discloses β2 agonists of formula :

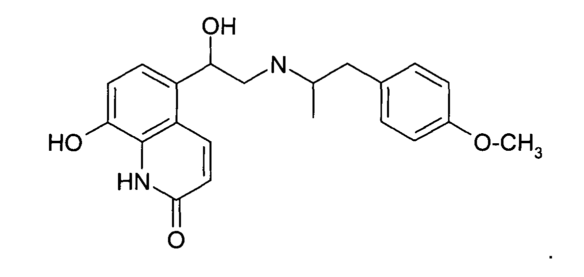

Other quinolinone derivatives are also disclosed in WO02/06235 as β3 agonists They are more specifically of formula :

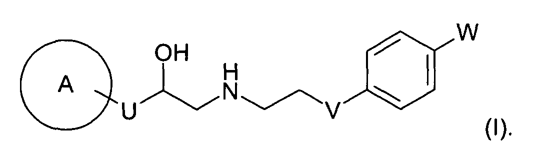

However, none of the above quinolinone derivatives have shown a pharmacological profile allowing them to be used as efficient drugs in the treatment of β2-mediated diseases and/or conditions, such as allergic and non-allergic airways diseases, in particular by the inhalation route.

The invention relates to the compounds of general formula (1): 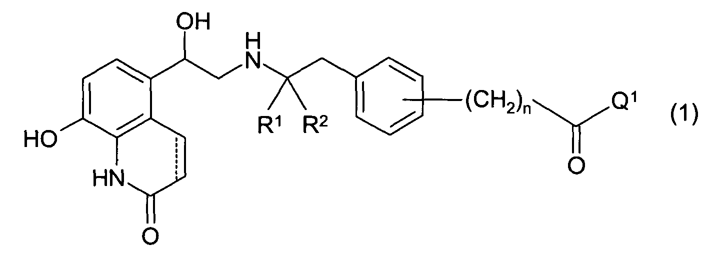 wherein the dashed line represents an optional bond, the (CH₂)ₙ-C(=O)Q¹ group is in the meta or para position,
- R¹ and R² are independently selected from H and C₁-C₄ alkyl,
- n is 0, 1 or 2, and,
- Q¹ is a group selected from: 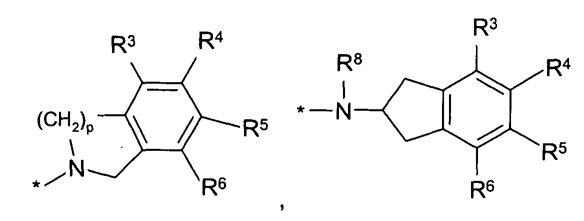 and a group *-NR⁸-Q²-A, wherein p is 1 or 2, Q² is a C₁-C₄ alkylene, R⁸ is H or C₁-C₄ alkyl and A is pyridyl, C₃-C₇ cycloalkyl, tetrahydropyranyl, piperidinyl, tetrahydrothiopyranyl or a group 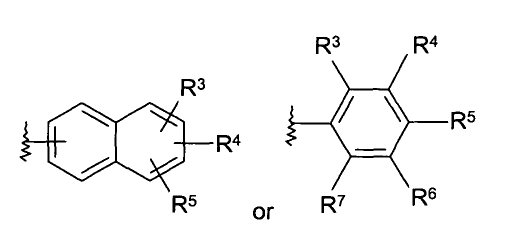
- R³, R⁴, R⁵, R⁶ and R⁷ are the same or different and are selected from H, C₁-C₄ alkyl, OR⁹, SR⁹, halo, CF₃, OCF₃, SO₂NR⁹R¹⁰, CONR⁹R¹⁰, NR⁹R¹⁰, NHCOR¹¹ and phenyl;
- R¹¹ is C₁-C₄ alkyl and R⁹ and R¹⁰ are the same or different and are selected from H or C₁-C₄ alkyl and the * represent the attachment point to the carbonyl group;
or, if appropriate, their pharmaceutically acceptable salts and/or isomers, tautomers, solvates or isotopic variations thereof.

The compounds of formula (1) are agonists of the β2 receptors, that are particularly useful for the treatment of β2-mediated diseases and/or conditions, by showing excellent potency, in particular when administered via the inhalation route.

In the here above general formula (1), C₁-C₄ alkyl and C₁-C₄ alkylene denote a straight-chain or branched group containing 1, 2, 3 or 4 carbon atoms. This also applies if they carry substituents or occur as substituents of other radicals, for example in O-(C₁-C₄)alkyl radicals, S-(C₁-C₄)alkyl radicals etc.... Examples of suitable (C₁-C₄)alkyl radicals are methyl, ethyl, *n*-propyl, *iso*-propyl, *n*-butyl, *iso*-butyl, *sec*-butyl, *tert*-butyl.... Examples of suitable O-(C₁-C₄)alkyl radicals are methoxy, ethoxy, *n*-propyloxy, *iso*-propyloxy, *n*-butyloxy, *iso*-butyloxy, *sec*-butyloxy and *tert*-butyloxy....

The term "C₃-C₇ cycloalkyl", includes cyclopropyl, cyclobutyl, cyclopentyl, cyclohexyl and cycloheptyl. A preferred cycloalkyl group is cyclohexyl.

Finally, halo denotes a halogen atom selected from the group consisting of fluoro, chloro, bromo and iodo in particular fluoro or chloro.

In the following, the free bond on the phenyl group such as in the structure below, 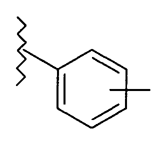 means that the phenyl can be substituted in the meta or para position.

In the above and in the following, the dashed line the represents an optional bond.

The compounds of the formula (1) 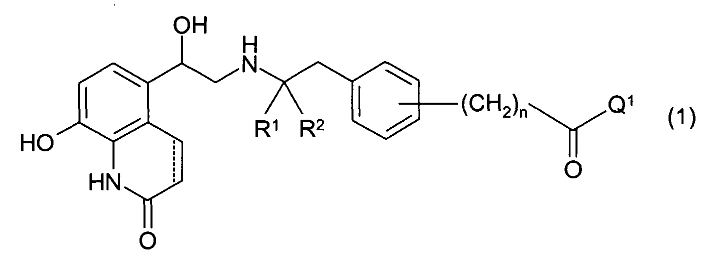 can be prepared using conventional procedures such as by the following illustrative methods in which R¹, R², Q¹, and n are as previously defined for the compounds of the formula (1) unless otherwise stated.

The compounds of the formula (1) may be prepared by deprotection of the protected compound of formula (2): 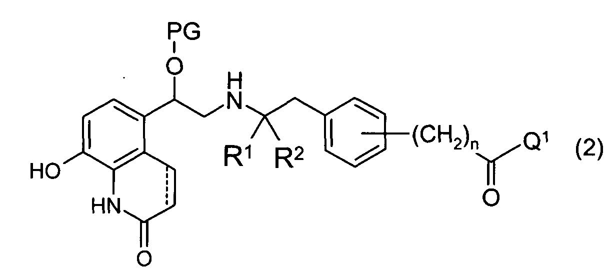 wherein R¹, R², Q¹, and n are as previously defined and PG represents a suitable alcohol protecting group, typically a silyl group such as TBDMS or TMS, and preferably TBDMS.
The deprotection may be carried out according to the methods described in standard text-books such as "Protective Groups in Organic Synthesis" by T.W.Greene, A.Wiley-Interscience Publication, 1981. In a typical procedure, where PG represents TBDMS, compound of formula (2) is treated with 10-18 eq ammonium fluoride in aqueous methanol, at about 45°C for between 18 and 42 hours.

The amide derivatives of formula (2) may be prepared by coupling an acid of formula (3): 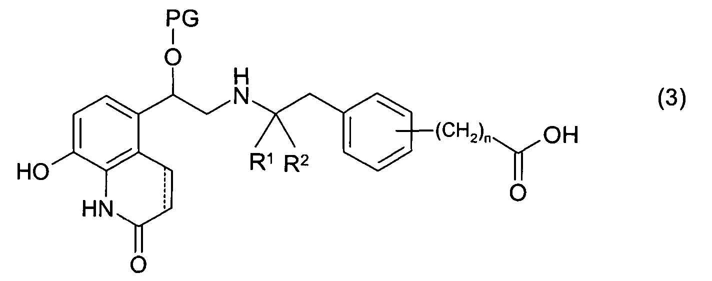 with an amine of formula -NHR⁸-Q²-A (4),

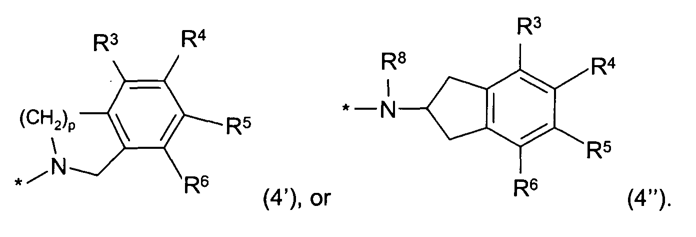

The coupling is generally carried out in an excess of said amine as an acid receptor, with a conventional coupling agent (e.g. 1-(3-dimethylaminopropyl)-3-ethylcarbodiimide hydrochloride or *N*, *N*'-dicyclohexylcarbodiimide), optionally in the presence of a catalyst (e.g. 1-hydroxybenzotriazole hydrate or 1-hydroxy-7-azabenzotriazole), and optionally in the presence of a tertiary amine base (e.g. *N*-methylmorpholine, triethylamine or diisopropylethylamine). The reaction may be undertaken in a suitable solvent such as pyridine, dimethylformamide, tetrahydrofuran, dimethylsulfoxide, dichloromethane or ethyl acetate, and at temperature comprised between 10°C and 40°C (room temperature) for a period of 1-24 hours.

In a typical procedure, the acid of formula (3) is treated with an excess of amine of formula (4), (4') or (4") (1.2-2.1 eq), 1-(3-dimethylaminopropyl)-3-ethylcarbodiimide hydrochloride (1.2-1.4 eq), 1-hydroxybenzotriazole hydrate (1.1-1.4eq) and triethylamine (2-3 eq) in N,N-dimethylformamide for a period of 18 hours.

Said amine (4), (4') or (4") is either commercially available or may be prepared by conventional methods well known to the one skilled in the art (e.g. reduction, oxidation, alkylation, transition metal-mediated coupling, protection, deprotection etc...) from commercially available material.

The acid of formula (3) may be prepared from the corresponding ester of formula (5) : 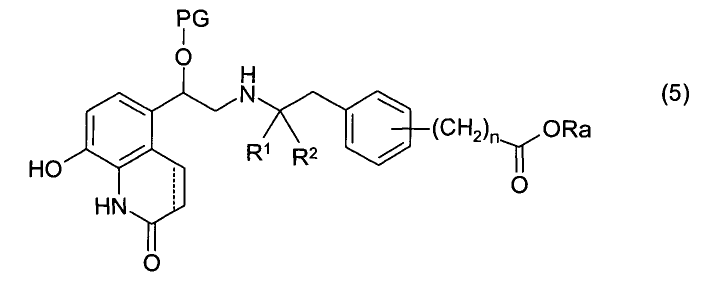 wherein Ra is a suitable acid protecting group, typically benzyl or a (C₁-C₄)alkyl group and preferably a (C₁-C₄)alkyl group, which includes, but is not limited to, methyl and ethyl, according to any method well-known to the one skilled in the art to prepare an acid from an ester, without modifying the rest of the molecule. For example, the ester may be hydrolysed by treatment with aqueous acid or base (e.g. hydrogen chloride, potassium hydroxide, sodium hydroxide or lithium hydroxide), optionally in the presence of a solvent or mixture of solvents (e.g. water, 1,4-dioxan, tetrahydrofuran/water), at a temperature comprised between 20°C and 100°C, for a period of 1 to 40 hours.

In a typical procedure the ester of formula (5) is treated with an excess of lithium or sodium hydroxide (3-5eq) in aqueous dioxan or tetrahydrofuran for about 18 hours.

The ester of formula (5) may be prepared by deprotection of the compound of formula (6): 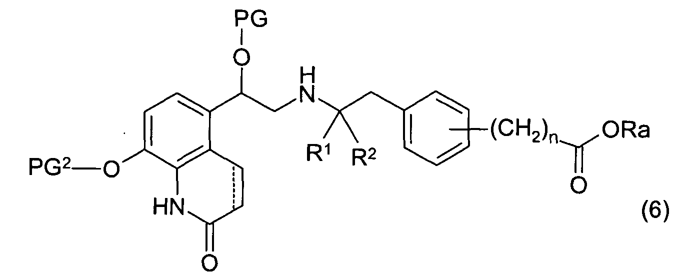 wherein PG² represents a suitable alcohol protecting group, typically benzyl or acetate, and preferably benzyl.

The deprotection may be carried out according to the methods described in standard text-books such as "Protective Groups in Organic Synthesis" by T.W.Greene, A.Wiley-Interscience Publication, 1981.

In a typical procedure, wherein PG² is benzyl, compound of formula (6) is hydrogenated in the presence of 10% palladium on charcoal in ethanol or methanol as solvent at 60 psi of H₂, for up to 40 hours at room temperature.

Alternatively, compound of formula (6) is treated with 20% palladium on charcoal, an excess of ammonium formate in ethanol at 80°C for about 1 hours.

The compound of formula (6) may be prepared by reaction of an amine of formula (7) : 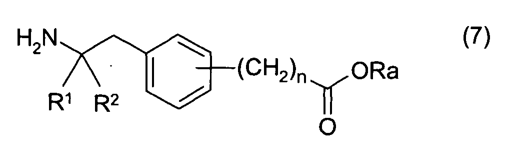 wherein Ra, R¹, R² and n are as previously defined, with a bromide of formula (8) : 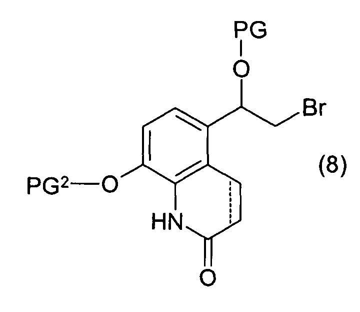 wherein PG and PG² are as previously defined.In a typical procedure, the amine of formula (7) is reacted with a bromide of formula (8) at 90°C for between 18 and 48 hours, in the absence of solvent.

The bromide of formula (8) may be prepared by analogy with the methods of Iwakuma et. al. (EP 147719) or Moran et. al. (WO 03/042160).

When one of R¹ and R² represents C₁-C₄ alkyl and the other is H, the amine of formula (7) may be prepared as either the (*R*) or (*S*) enantiomer from the corresponding protected amine of formula (9) : 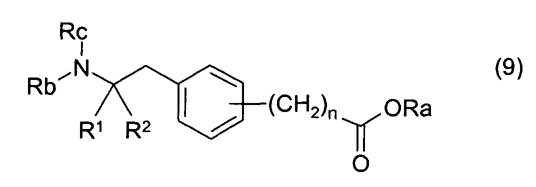 wherein Ra, R¹, R² and n are as previously defined and Rb and Rc represent any suitable substituents so that HNRbRc is a chiral amine (for example, Rb may be hydrogen and Rc may be α-methylbenzyl), provided that the bonds between N and Rb and N and Rc can be easily cleaved to give the free amine of formula (7) using standard methodology for cleaving nitrogen protecting groups, such as those found in the text book T.W. GREENE, Protective Groups in Organic Synthesis , A. Wiley-Interscience Publication, 1981.

In a typical procedure, the amine of formula (9) is treated with 20% palladium hydroxide on charcoal and an excess of ammonium formate in ethanol at room temperature for about 2 hours.

The amine of formula (9) may be prepared as a single diastereomer by reaction of an amine of formula HNRbRc with a ketone of formula (10): 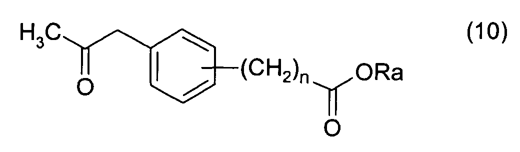 wherein Ra, Rb, Rc and n are as previously defined.

In a typical procedure, the reaction of the ketone of formula (10) with the amine of formula HNRbRc leads to a chiral intermediate which is in turn reduced by a suitable reducing agent (e.g. sodium cyanoborohydride of formula NaCNBH₃ or sodium triacetoxyborohydride of formula Na(OAc)₃BH) optionally in the presence of a drying agent (e.g. molecular sieves, magnesium sulfate) and optionally in the presence of an acid catalyst (e.g. acetic acid) to give the amine of formula (9) as a mixture of diastereomers. The reaction is generally performed in a solvent such as tetrahydrofuran or dichloromethane at a temperature comprised between 20°C and 80°C for 3 to 72 hours. The resulting product is then converted to the hydrochloride salt and selectively crystallised from a suitable solvent or mixture of solvents (e.g. isopropanol, ethanol, methanol, diisopropyl ether or diisopropyl ether/methanol) to give (9) as a single diastereomer.

The ketone of formula (10) may be prepared by palladium mediated coupling of an aryl halide of formula (11): 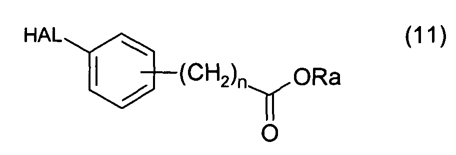 wherein Ra and n are as previously defined and HAL represents a halogen atom, which includes, but is not limited to bromo and iodo, with an enolate or enolate equivalent.

In a typical procedure, the aryl halide of formula (11) is reacted with a tin enolate generated in-situ by treatment of isopropenyl acetate with tri-n-butyltin methoxide of formula Bu₃SnOMe in the presence of a suitable palladium catalyst (palladium acetate/ tri-*ortho*-tolylphosphine of formula Pd(OAc)₂/P(o-Tol)₃) in a non-polar solvent (e.g. toluene, benzene, hexane). Preferably, the reaction is carried out at a temperature comprised between 80°C and 110°C for 5 to 16 hours.

The aryl halide of formula (11) may be obtained by esterification of the corresponding acid of formula (12): 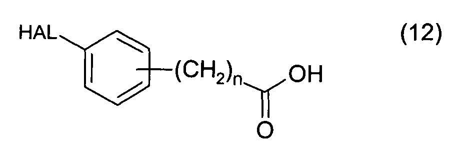 wherein HAL is as previously defined, according to any method well-known to the one skilled in the art to prepare an ester from an acid, without modifying the rest of the molecule.

In a typical procedure, the acid of formula (12) is reacted with an alcoholic solvent of formula RaOH, wherein Ra is as previously defined, in the presence of an acid such as hydrogen chloride at a temperature between 10°C and 40°C (room temperature) for upto 16 hours.

The acid of formula (12) is a commercial product.

When R¹ and R² both represent C₁-C₄ alkyl, then the amine of formula (7) may be prepared from the compound of formula (13): 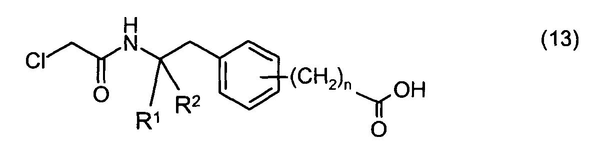 by cleavage of the chloroacetyl group, by analogy with the method of Jirgensons et. al. Synthesis, 2000, 1709, followed by in-situ esterification in the presence of RaOH under acidic conditions, as described previously.

In a typical procedure, the amide of formula (13) is treated with a small excess of thiourea in acetic acid and ethanol at the reflux temperature of the reaction for about 16 hours, followed by treatment of the product with ethanolic hydrogen chloride for 16 hours at the reflux temperature of the reaction.

The amide of formula (13) may be prepared from the alcohol of formula (14): 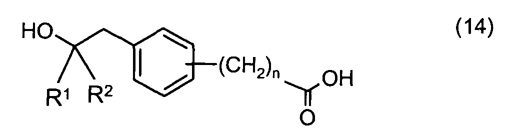 by reaction with chloroacetonitrile (ClCH₂CN), by analogy with the methods of Jirgensons et. al. Synthesis 2000, 1709 or Krimmen et. al. Organic Reactions 1969,17,213.

In a typical procedure the alcohol of formula (14) is treated with an excess of chloroacetonitrile in acetic acid and sulphuric acid at between 0°C and room temperature for about 5 hours.

The alcohol of formula (14) may be prepared from the ester of formula (15): 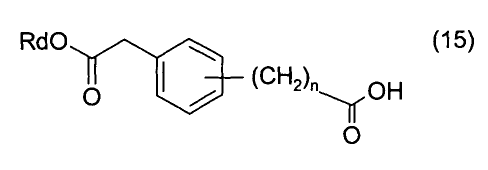 wherein n is as previously defined and Rd represents a suitable acid protecting group, typically benzyl or a (C₁-C₄) alkyl group, which includes, but is not limited to, methyl or ethyl, according to any method well-known to the one skilled in the art to prepare an acid from an ester, without modifying the rest of the molecule. The ester is reacted with a suitable organometallic reagent, (e.g. R²Li, R²MgBr) in a suitable solvent such as tetrahydrofuran or ether.

In a typical procedure, the ester of formula (15) is treated with an excess of CH₃MgBr in ether at between 0°C and room temperature for about 2 hours.

The acid of formula (15) may be prepared by partial hydrolysis of the diester of formula (16): 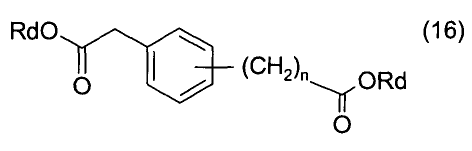 where Rd is as previously defined, and are preferably the same, by analogy with the method of Vander Elst et. al. Int. J. Peptide Protein Res. 29, 1987, 331-346.

In a typical procedure, the diester of formula (16) is treated with ethanolic hydrochloric acid in dioxan in the presence of 2 eq of the diacid of formula (17), at the reflux temperature of the reaction for 18 hours.

The diester of formula (16) may be prepared by esterification of the corresponding diacid of formula (17): 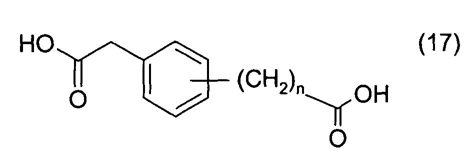 according to any method well-known to the one skilled in the art to prepare an ester from an acid, without modifying the rest of the molecule.

In a typical procedure, the acid of formula (17) is treated with catalytic acetyl chloride in an alcoholic solvent of formula RdOH, wherein Rd is as previously defined, at the reflux temperature of the reaction for about 18 hours.

The acid of formula (17) is commercially available.

Alternatively, the amine of formula (7), where R¹ = R² = C₁-C₄ alkyl and n=0, may be prepared according to the following scheme: 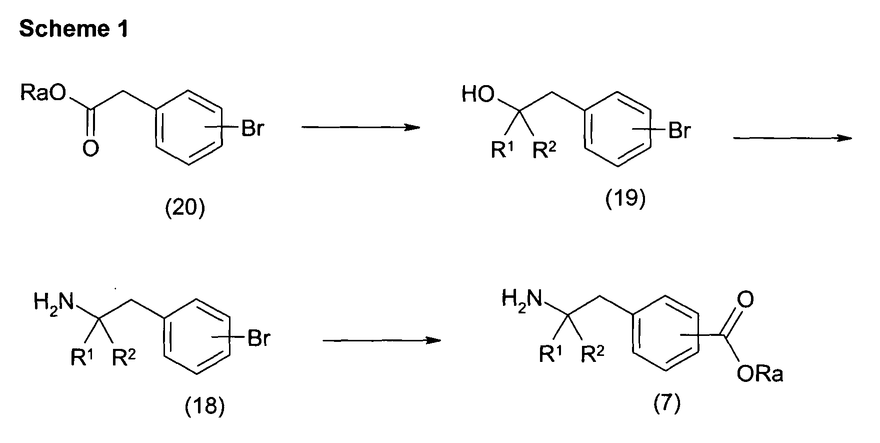 wherein Ra are as previously defined.

In a typical procedure, the ester of formula (20) is reacted with an "activated" alkyl (organometallic alkyl such as R²MgBr, R²MgC, or R²Li) to give the corresponding tertiary alcohol of formula (19) using the method described above.

Said tertiary alcohol of formula (19) is then treated with an alkyl nitrile (e.g. acetonitrile, chloroacetonitrile) in the presence of an acid (e.g. sulphuric acid, acetic acid) to give a protected intermediate which is in turn cleaved using standard methodology for cleaving nitrogen protecting group such as those mentioned in textbooks to give the bromo amine (18).

The resulting bromo amine (18) is treated with a suitable palladium catalyst (e.g. [1,1'-bis(diphenylphophino)ferrocene]dichloropalladium(II)) under an atmosphere of carbon monoxide using RaOH as solvent (e.g. MeOH, EtOH) at elevated temperature (100°C) and pressure (100psi) to give the ester of formula (7).

Alternatively, The ketone of formula (10) where n=2 may be prepared by reduction of an alkene of formula (21) :

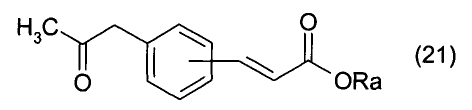

In a typical procedure, a solution of the olefin of formula (21) in a suitable solvent (e.g. methanol, ethanol, ethyl acetate) is treated with a palladium catalyst (e.g. 10% palladium on charcoal) and stirred under an atmosphere of hydrogen, optionally at elevated pressure (e.g. 60 psi), at temperature between room temperature and 60°C for 8-24 hours.

The alkene of formula (21) may be prepared by a palladium mediated coupling of an activated olefin with an aryl halide of formula (22):

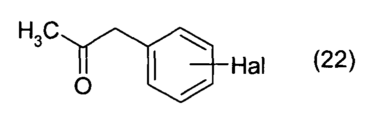

In a typical procedure, the aryl halide (22) is coupled with a vinyl ester (e.g. methyl acrylate) in the presence of a suitable palladium catalyst (e.g. tetrakis(triphenylphosphine)palladium(0) of formula Pd(PPh₃)₄, palladium acetate/tri-*ortho*-tolylphosphine of formula Pd(OAc)₂/P(o-tol)₃ or (diphenylphosphino)ferrocenyl palladium chloride of formula dppfPdCl₂) in a sutiable solvent (e.g. acetonitrile, *N,N-*dimethylformamide, toluene), optionally in the presence of a base such as triethylamine at a temperature between 40°C and 110°C for 8 to 24 hours.

The ketone of formula (22) is a commercial product.

The compounds of formula (1) wherein the dashed line is a bond can be reduced to a compound of formula (1) wherein the dashed line doesn't represent a bond, according to processes well known to the man skilled in the art. For example, it may be hydrogenated using 10% palladium on carbon catalyst at 30°C for 48 hours under one atmosphere of hydrogen.

For some of the steps of the here above described process of preparation of the compounds of formula (1), it may be necessary to protect potential reactive functions that are not wished to react, and to cleave said protecting groups in consequence. In such a case, any compatible protecting radical can be used. In particular methods of protection and deprotection such as those described by T.W. GREENE (*Protective Groups in Organic Synthesis,* A. Wiley-Interscience Publication, 1981) or by P. J. Kocienski (*Protecting groups,* Georg Thieme Verlag, 1994), can be used.

All of the above reactions and the preparations of novel starting materials used in the preceding methods are conventional and appropriate reagents and reaction conditions for their performance or preparation as well as procedures for isolating the desired products will be well-known to those skilled in the art with reference to literature precedents and the examples and preparations hereto.

Also, the compounds of formula (1) as well as intermediate for the preparation thereof can be purified according to various well-known methods, such as for example crystallization or chromatography.

All of the above reactions and the preparations of novel starting materials used in the preceding methods are conventional and appropriate reagents and reaction conditions for their performance or preparation as well as procedures for isolating the desired products will be well-known to those skilled in the art with reference to literature precedents and the examples and preparations hereto.

Also, the compounds of formula (1) as well as intermediate for the preparation thereof can be purified according to various well-known methods, such as for example crystallization or chromatography.

In the compounds of formula (1), the following substituents are preferred:

Preferably, Q¹ is a group *-NH-Q²-A, wherein A is cyclohexyl or cycloheptyl.

Preferably, A is adamantyl.Preferably, Q¹ is 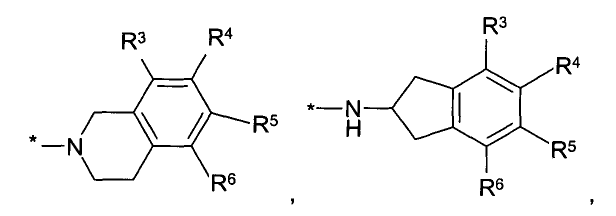 wherein R₃, R₄, R₅ and R₆ are H.

Preferably, Q¹ is a group *-NH-Q²-A, wherein A is a group 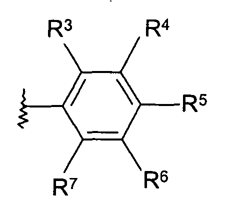 wherein R³, R⁴, R⁵, R⁶ and R⁷ are the same or different and are selected from H, C₁-C₄ alkyl, OR⁹, SR⁹, halo, CF₃, OCF₃, SO₂NR⁹R¹⁰, CONR⁹R¹⁰, NR⁹R¹⁰, NHCOR¹¹ and phenyl provided at least 2 of R³ to R⁷ are equal to H;
wherein R¹¹ is C₁-C₄ alkyl and R⁹ and R¹⁰ are the same or different and are selected from H or C₁-C₄ alkyl.

More preferably, Q¹ is a group *-NH-Q²-A, wherein A is a group 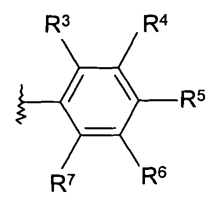 wherein R³, R⁴, R⁵, R⁶ and R⁷ are the same or different and are selected from H, CH₃, OCH₃, OCH₂-CH₃, SCH₃, halo, CF₃, provided at least 2 of R³ to R⁷ are equal to H.

In the above groups of compounds, the following substituents are particularly preferred:
Q² is -CH₂-, -(CH₂)₂-, -(CH₂)₃-, or (CH(CH₃)₂)-, preferably -CH₂-.
R¹ is H or C₁-C₄ alkyl and R² is C₁-C₄ alkyl. More preferably, R¹ is H or CH₃ and R² is CH₃ or CH₂CH₃.
n is 1.
R¹ is H and R² is CH₃ or CH₂CH₃ and n is 1.
R¹ is CH₃, R² is CH₃ and n is 1.

Particularly preferred are the compounds of the formula (1) as described in the Examples section hereafter, i.e. :
*N*-(cyclohexylmethyl)-2-[4-((2*R*)-2-{[(2*R*)-2-hydroxy-2-(8-hydroxy-2-oxo-1,2-dihydroquinolin-5-yl)ethyl]amino}propyl)phenyl]acetamide;
5-{(1*R*)-2-[((1*R*)-2-{4-[2-(3,4-dihydroisoquinolin-2(1*H*)-yl)-2-oxoethyl]phenyl}-1-methylethyl)amino]-1-hydroxyethyl}-8-hydroxyquinolin-2(1*H*)-one;
*N*-benzyl-2-[4-((2*R*)-2-{[(2*R*)-2-hydroxy-2-(8-hydroxy-2-oxo-1,2-dihydroquinolin-5-yl)ethyl]amino}propyl)phenyl]acetamide;
2-[4-((2*R*)-2-{[(2*R*)-2-hydroxy-2-(8-hydroxy-2-oxo-1,2-dihydroquinolin-5-yl)ethyl]amino}propyl)phenyl]-*N*-(2-methoxybenzyl)acetamide;
*N*-(4-chlorobenzyl)-2-[4-((2*R*)-2-{[(2*R*)-2-hydroxy-2-(8-hydroxy-2-oxo-1,2-dihydroquinolin-5-yl)ethyl]amino}propyl)phenyl]acetamide;
2-[4-((2*R*)-2-{[(2*R*)-2-hydroxy-2-(8-hydroxy-2-oxo-1,2-dihydroquinolin-5-yl)ethyl]amino}propyl)phenyl]-*N*-[3-(trifluoromethyl)benzyl]acetamide;
*N*-(2,6-dimethoxybenzyl)-2-[4-((2*R*)-2-{[(2*R*)-2-hydroxy-2-(8-hydroxy-2-oxo-1,2-dihydroquinolin-5-yl)ethyl]amino}propyl)phenyl]acetamide;
*N*-[2-fluoro-5-(trifluoromethyl)benzyl]-2-[4-((2*R*)-2-{[(2*R*)-2-hydroxy-2-(8-hydroxy-2-oxo-1,2-dihydroquinolin-5-yl)ethyl]amino}propyl)phenyl]acetamide;
*N*-(3,4-dichlorobenzyl)-2-[4-((2*R*)-2-{[(2*R*)-2-hydroxy-2-(8-hydroxy-2-oxo-1,2-dihydroquinolin-5-yl)ethyl]amino}propyl)phenyl]acetamide;
2-[4-((2*R*)-2-{[(2*R*)-2-hydroxy-2-(8-hydroxy-2-oxo-1,2-dihydroquinolin-5-yl)ethyl]amino}propyl)phenyl]-*N*-(3-phenylpropyl)acetamide;
*N*-(cyclohexylmethyl)-2-[3-((2*R*)-2-{[(2*R*)-2-hydroxy-2-(8-hydroxy-2-oxo-1,2-dihydroquinolin-5-yl)ethyl]amino}propyl)phenyl]acetamide;
*N*-benzyl-2-[3-((2*R*)-2-{[(2*R*)-2-hydroxy-2-(8-hydroxy-2-oxo-1,2-dihydroquinolin-5-yl)ethyl]amino}propyl)phenyl]acetamide;
*N*-(2-ethoxybenzyl)-2-[3-((2*R*)-2-{[(2*R*)-2-hydroxy-2-(8-hydroxy-2-oxo-1,2-dihydroquinolin-5-yl)ethyl]amino}propyl)phenyl]acetamide;
*N*-(3,4-dimethylbenzyl)-2-[3-((2*R*)-2-{[(2*R*)-2-hydroxy-2-(8-hydroxy-2-oxo-1,2-dihydroquinolin-5-yl)ethyl]amino}propyl)phenyl]acetamide;
*N*-(2-chloro-6-methylbenzyl)-2-[3-((2*R*)-2-{[(2*R*)-2-hydroxy-2-(8-hydroxy-2-oxo-1,2-dihydroquinolin-5-yl)ethyl]amino}propyl)phenyl]acetamide;
*N*-(3-chloro-4-methylbenzyl)-2-[3-((2*R*)-2-{[(2*R*)-2-hydroxy-2-(8-hydroxy-2-oxo-1,2-dihydroquinolin-5-yl)ethyl]amino}propyl)phenyl]acetamide;
*N*-(3,4-dichlorobenzyl)-2-[3-((2*R*)-2-{[(2*R*)-2-hydroxy-2-(8-hydroxy-2-oxo-1,2-dihydroquinolin-5-yl)ethyl]amino}propyl)phenyl]acetamide;
*N*-[2-fluoro-5-(trifluoromethyl)benzyl]-2-[3-((2*R*)-2-{[(2*R*)-2-hydroxy-2-(8-hydroxy-2-oxo-1,2-dihydroquinolin-5-yl)ethyl]amino}propyl)phenyl]acetamide;
*N*-[2-(4-chlorophenyl)ethyl]-2-[3-((2*R*)-2-{[(2*R*)-2-hydroxy-2-(8-hydroxy-2-oxo-1,2-dihydroquinolin-5-yl)ethyl]amino}propyl)phenyl]acetamide;
2-[3-((2*R*)-2-{[(2*R*)-2-hydroxy-2-(8-hydroxy-2-oxo-1,2-dihydroquinolin-5-yl)ethyl]amino}propyl)phenyl]-*N*-(3-phenylpropyl)acetamide;
*N*-(cyclohexylmethyl)-2-[3-(2-{[(2*R*)-2-hydroxy-2-(8-hydroxy-2-oxo-1,2-dihydroquinolin-5-yl)ethyl]amino}-2-methylpropyl)phenyl]acetamide;
*N*-(cyclohexylmethyl)-*N*-ethyl-2-[3-(2-{[(2*R*)-2-hydroxy-2-(8-hydroxy-2-oxo-1,2-dihydroquinolin-5-yl)ethyl]amino}-2-methylpropyl)phenyl]acetamide;
*N*-(3,4-dimethylbenzyl)-2-[3-(2-{[(2*R*)-2-hydroxy-2-(8-hydroxy-2-oxo-1,2-dihydroquinolin-5-yl)ethyl]amino}-2-methylpropyl)phenyl]acetamide;
*N*-(2-chloro-6-methylbenzyl)-2-[3-(2-{[(2*R*)-2-hydroxy-2-(8-hydroxy-2-oxo-1,2-dihydroquinolin-5-yl)ethyl]amino}-2-methylpropyl)phenyl]acetamide;
*N*-(3,4-dichlorobenzyl)-2-[3-(2-{[(2*R*)-2-hydroxy-2-(8-hydroxy-2-oxo-1,2-dihydroquinolin-5-yl)ethyl]amino}-2-methylpropyl)phenyl]acetamide;
*N*-(3,5-dichlorobenzyl)-2-[3-(2-{[(2*R*)-2-hydroxy-2-(8-hydroxy-2-oxo-1,2-dihydroquinolin-5-yl)ethyl]amino}-2-methylpropyl)phenyl]acetamide;
*N*-[2-fluoro-5-(trifluoromethyl)benzyl]-2-[3-(2-{[(2*R*)-2-hydroxy-2-(8-hydroxy-2-oxo-1,2-dihydroquinolin-5-yl)ethyl]amino}-2-methylpropyl)phenyl]acetamide;
2-[3-(2-{[(2*R*)-2-hydroxy-2-(8-hydroxy-2-oxo-1,2-dihydroquinolin-5-yl)ethyl]amino}-2-methylpropyl)phenyl]-*N*-[2-(methylthio)benzyl]acetamide;
2-[3-(2-{[(2*R*)-2-hydroxy-2-(8-hydroxy-2-oxo-1,2-dihydroquinolin-5-yl)ethyl]amino}-2-methylpropyl)phenyl]-*N*-[4-(methylthio)benzyl]acetamide;
*N*-[2-(4-chlorophenyl)ethyl]-2-[3-(2-{[(2*R*)-2-hydroxy-2-(8-hydroxy-2-oxo-1,2-dihydroquinolin-5-yl)ethyl]amino}-2-methylpropyl)phenyl]acetamide, and,
2-[3-(2-{[(2*R*)-2-hydroxy-2-(8-hydroxy-2-oxo-1,2-dihydroquinolin-5-yl)ethyl]amino}-2-methylpropyl)phenyl]-*N*-(3-phenylpropyl)acetamide.

According to one aspect of the present invention, the compounds of formula (1) wherein the (CH₂)ₙ-C(=O)Q¹ group is in the meta position are generally preferred.

According to one aspect of the present invention, the compounds of formula (1) wherein the dashed line represents a bond, such as in the formula below, are preferred:

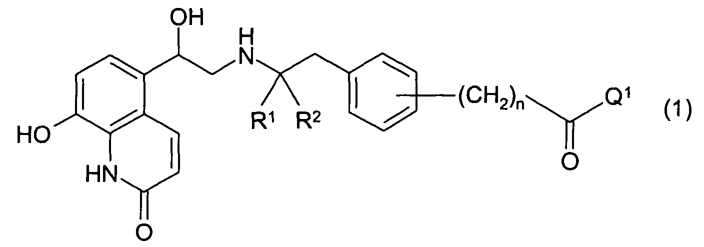

Pharmaceutically acceptable salts of the compounds of formula (1) include the acid addition and base salts thereof.

Suitable acid addition salts are formed from acids which form non-toxic salts. Examples include the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.
Hemisalts of acids and bases may also be formed, for example, hemisulphate and hemicalcium salts.

For a review on suitable salts, see "Handbook of Pharmaceutical Salts: Properties, Selection, and Use" by Stahl and Wermuth (Wiley-VCH, Weinheim, Germany, 2002).

Pharmaceutically acceptable salts of compounds of formula (1) may be prepared by one or more of three methods:
(i) by reacting the compound of formula (1) with the desired acid or base;
(ii) by removing an acid- or base-labile protecting group from a suitable precursor of the compound of formula (1) or by ring-opening a suitable cyclic precursor, for example, a lactone or lactam, using the desired acid or base; or
(iii) by converting one salt of the compound of formula (1) to another by reaction with an appropriate acid or base or by means of a suitable ion exchange column.

All three reactions are typically carried out in solution. The resulting salt may precipitate out and be collected by filtration or may be recovered by evaporation of the solvent. The degree of ionisation in the resulting salt may vary from completely ionised to almost non-ionised.

The compounds of the invention may exist in both unsolvated and solvated forms. The term 'solvate' is used herein to describe a molecular complex comprising the compound of the invention and a stoichiometric amount of one or more pharmaceutically acceptable solvent molecules, for example, ethanol. The term 'hydrate' is employed when said solvent is water.

Included within the scope of the invention are complexes such as clathrates, drug-host inclusion complexes wherein, in contrast to the aforementioned solvates, the drug and host are present in stoichiometric or non-stoichiometric amounts. Also included are complexes of the drug containing two or more organic and/or inorganic components which may be in stoichiometric or non-stoichiometric amounts. The resulting complexes may be ionised, partially ionised, or non-ionised. For a review of such complexes, see J Pharm Sci, 64 (8), 1269-1288 by Haleblian (August 1975).

Hereinafter all references to compounds of formula (1) include references to salts, solvates and complexes thereof and to solvates and complexes of salts thereof.

The compounds of the invention include compounds of formula (1) as hereinbefore defined, including all polymorphs and crystal habits thereof, prodrugs and isomers thereof (including optical, geometric and tautomeric isomers) as hereinafter defined and isotopically-labeled compounds of formula (1).

As indicated, so-called 'pro-drugs'_of the compounds of formula (1) are also within the scope of the invention. Thus certain derivatives of compounds of formula (1) which may have little or no pharmacological activity themselves can, when administered into or onto the body, be converted into compounds of formula (1) having the desired activity, for example, by hydrolytic cleavage. Such derivatives are referred to as 'prodrugs'. Further information on the use of prodrugs may be found in 'Pro-drugs as Novel Delivery Systems, Vol. 14, ACS Symposium Series (T. Higuchi and W. Stella) and 'Bioreversible Carriers in Drug Design', Pergamon Press, 1987 (ed. E. B Roche, American Pharmaceutical Association).

Prodrugs in accordance with the invention can, for example, be produced by replacing appropriate functionalities present in the compounds of formula (1) with certain moieties known to those skilled in the art as 'pro-moieties' as described, for example, in "Design of Prodrugs" by H. Bundgaard (Elsevier, 1985).

Some examples of prodrugs in accordance with the invention include:
(i) where the compound of formula (1) contains a carboxylic acid functionality (-COOH), an ester thereof, for example, a compound wherein the hydrogen of the carboxylic acid functionality of the compound of formula (1) is replaced by (C₁-C₈)alkyl;
(ii) where the compound of formula (1) contains an alcohol functionality (-OH), an ether thereof, for example, a compound wherein the hydrogen of the alcohol functionality of the compound of formula (1) is replaced by (C₁-C₆)alkanoyloxymethyl; and
(iii) where the compound of formula (1) contains a primary or secondary amino functionality (-NH₂ or -NHR where R ≠H), an amide thereof, for example, a compound wherein, as the case may be, one or both hydrogens of the amino functionality of the compound of formula (1) is/are replaced by (C₁-C₁₀)alkanoyl.

Further examples of replacement groups in accordance with the foregoing examples and examples of other prodrug types may be found in the aforementioned references.

Moreover, certain compounds of formula (1) may themselves act as prodrugs of other compounds of formula (1).
Also included within the scope of the invention are metabolites of compounds of formula (1), that is, compounds formed *in vivo* upon administration of the drug. Some examples of metabolites in accordance with the invention include
(i) where the compound of formula (1) contains a methyl group, an hydroxymethyl derivative thereof (-CH₃ -> -CH₂OH):
(ii) where the compound of formula (1) contains an alkoxy group, an hydroxy derivative thereof (-OR -> -OH);
(iii) where the compound of formula (1) contains a tertiary amino group, a secondary amino derivative thereof (-NR¹R² -> -NHR¹ or -NHR²);
(iv) where the compound of formula (1) contains a secondary amino group, a primary derivative thereof (-NHR¹ -> -NH₂);
(v) where the compound of formula (1) contains a phenyl moiety, a phenol derivative thereof (-Ph -> -PhOH); and
(vi) where the compound of formula (1) contains an amide group, a carboxylic acid derivative thereof (-CONH₂ -> COOH).

Compounds of formula (1) containing one or more asymmetric carbon atoms can exist as two or more stereoisomers. Where a compound of formula (1) contains an alkenyl or alkenylene group, geometric *cis*/*trans* (or Z/E) isomers are possible. Where structural isomers are interconvertible *via* a low energy barrier, tautomeric isomerism ('tautomerism') can occur. This can take the form of proton tautomerism in compounds of formula (1) containing, for example, an imino, keto, or oxime group, or so-called valence tautomerism in compounds which contain an aromatic moiety. It follows that a single compound may exhibit more than one type of isomerism.

Included within the scope of the present invention are all stereoisomers, geometric isomers and tautomeric forms of the compounds of formula (1), including compounds exhibiting more than one type of isomerism, and mixtures of one or more thereof. Also included are acid addition or base salts wherein the counterion is optically active, for example, *d*-lactate or *l*-lysine, or racemic, for example, *dl*-tartrate or *dl*-arginine.

*Cis*/*trans* isomers may be separated by conventional techniques well known to those skilled in the art, for example, chromatography and fractional crystallisation.

Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC).

Alternatively, the racemate (or a racemic precursor) may be reacted with a suitable optically active compound, for example, an alcohol, or, in the case where the compound of formula (1) contains an acidic or basic moiety, an acid or base such as tartaric acid or 1-phenylethylamine. The resulting diastereomeric mixture may be separated by chromatography and/or fractional crystallization and one or both of the diastereoisomers converted to the corresponding pure enantiomer(s) by means well known to a skilled person.

Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% by volume of isopropanol, typically from 2% to 20%, and from 0 to 5% by volume of an alkylamine, typically 0.1% diethylamine. Concentration of the eluate affords the enriched mixture.

Stereoisomeric conglomerates may be separated by conventional techniques known to those skilled in the art - see, for example, "Stereochemistry of Organic Compounds" by E. L. Eliel (Wiley, New York, 1994).

According to one aspect of the present invention, the (R,R)-stereoisomer of the formula below, wherein R¹ is hydrogen and R² is C₁-C₄ alkyl, preferably methyl, and n and Q¹ are as defined above, is generally preferred:

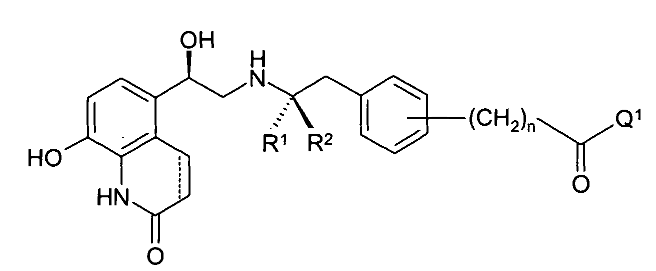

The present invention includes all pharmaceutically acceptable isotopically-labelled compounds of formula (1) wherein one or more atoms are replaced by atoms having the same atomic number, but an atomic mass or mass number different from the atomic mass or mass number which predominates in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S.

Certain isotopically-labelled compounds of formula (1), for example, those incorporating a radioactive isotope, are useful in drug and/or substrate tissue distribution studies. The radioactive isotopes tritium, *i*.*e*. ³H, and carbon-14, *i*.*e*. ¹⁴C, are particularly useful for this purpose in view of their ease of incorporation and ready means of detection.

Substitution with heavier isotopes such as deuterium, *i*.*e*. ²H, may afford certain therapeutic advantages resulting from greater metabolic stability, for example, increased *in vivo* half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds of formula (1) can generally be prepared by conventional techniques known to those skilled in the art or by processes analogous to those described in the accompanying Examples and Preparations using an appropriate isotopically-labeled reagents in place of the non-labeled reagent previously employed.

Pharmaceutically acceptable solvates in accordance with the invention include those wherein the solvent of crystallization may be isotopically substituted, e.g. D₂O, d₆-acetone, d₆-DMSO.

The compounds of formula (1), their pharmaceutically acceptable salts and/or derived forms, are valuable pharmaceutically active compounds, which are suitable for the therapy and prophylaxis of numerous disorders in which the β2 receptor is involved or in which agonism of this receptor may induce benefit, in particular the allergic and non-allergic airways diseases but also in the treatment of other diseases such as, but not limited to those of the nervous system, premature labor, congestive heart failure, depression, inflammatory and allergic skin diseases, psoriasis, proliferative skin diseases, glaucoma and in conditions where there is an advantage in lowering gastric acidity, particularly in gastric and peptic ulceration.

Compounds of the invention intended for pharmaceutical use may be administered as crystalline or amorphous products. They may be obtained, for example, as solid plugs, powders, or films by methods such as precipitation, crystallization, freeze drying, spray drying, or evaporative drying. Microwave or radio frequency drying may be used for this purpose.

They may be administered alone or in combination with one or more other compounds of the invention or in combination with one or more other drugs (or as any combination thereof). Generally, they will be administered as a formulation in association with one or more pharmaceutically acceptable excipients. The term "excipient" is used herein to describe any ingredient other than the compound(s) of the invention. The choice of excipient will to a large extent depend on factors such as the particular mode of administration, the effect of the excipient on solubility and stability, and the nature of the dosage form.

Pharmaceutical compositions suitable for the delivery of compounds of the present invention and methods for their preparation will be readily apparent to those skilled in the art. Such compositions and methods for their preparation may be found, for example, in 'Remington's Pharmaceutical Sciences', 19th Edition (Mack Publishing Company, 1995).
The compounds of the invention may be administered orally. Oral administration may involve swallowing, so that the compound enters the gastrointestinal tract, or buccal or sublingual administration may be employed by which the compound enters the blood stream directly from the mouth.

Formulations suitable for oral administration include solid formulations such as tablets, capsules containing particulates, liquids, or powders, lozenges (including
liquid-filled), chews, multi- and nano-particulates, gels, solid solution, liposome, films, ovules, sprays and liquid formulations.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, 11 (6), 981-986, by Liang and Chen (2001).

For tablet dosage forms, depending on dose, the drug may make up from 1 weight % to 80 weight % of the dosage form, more typically from 5 weight % to 60 weight % of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from 1 weight % to 25 weight %, preferably from 5 weight % to 20 weight % of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet.

Other possible ingredients include anti-oxidants, colourants, flavouring agents, preservatives and taste-masking agents.

Exemplary tablets contain up to about 80% drug, from about 10 weight % to about 90 weight % binder, from about 0 weight % to about 85 weight % diluent, from about 2 weight % to about 10 weight % disintegrant, and from about 0.25 weight % to about 10 weight % lubricant.

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

The formulation of tablets is discussed in Pharmaceutical Dosage Forms: Tablets, Vol. 1, by H. Lieberman and L. Lachman (Marcel Dekker, New York, 1980).

Consumable oral films for human or veterinary use are typically pliable water-soluble or water-swellable thin film dosage forms which may be rapidly dissolving or mucoadhesive and typically comprise a compound of formula (1), a film-forming polymer, a binder, a solvent, a humectant, a plasticiser, a stabiliser or emulsifier, a viscosity-modifying agent and a solvent. Some components of the formulation may perform more than one function.

The compound of formula (1) may be water-soluble or insoluble. A water-soluble compound typically comprises from 1 weight % to 80 weight %, more typically from 20 weight % to 50 weight %, of the solutes. Less soluble compounds may comprise a greater proportion of the composition, typically up to 88 weight % of the solutes. Alternatively, the compound of formula (1) may be in the form of multiparticulate beads.

The film-forming polymer may be selected from natural polysaccharides, proteins, or synthetic hydrocolloids and is typically present in the range 0.01 to 99 weight %, more typically in the range 30 to 80 weight %.

Other possible ingredients include anti-oxidants, colorants, flavourings and flavour enhancers, preservatives, salivary stimulating agents, cooling agents, co-solvents (including oils), emollients, bulking agents, anti-foaming agents, surfactants and taste-masking agents.

Films in accordance with the invention are typically prepared by evaporative drying of thin aqueous films coated onto a peelable backing support or paper. This may be done in a drying oven or tunnel, typically a combined coater dryer, or by freeze-drying or vacuuming.

Solid formulations for oral administration may be formulated to be immediate and/or modified release. Modified release formulations include delayed-, sustained-, pulsed-, controlled-, targeted and programmed release.

Suitable modified release formulations for the purposes of the invention are described in US Patent No. 6,106,864. Details of other suitable release technologies such as high energy dispersions and osmotic and coated particles are to be found in Pharmaceutical Technology On-line, 25(2), 1-14, by Verma *et al* (2001). The use of chewing gum to achieve controlled release is described in WO 00/35298.

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of compounds of formula (1) used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and poly(*dl*-lactic-coglycolic)acid (PGLA) microspheres.

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958 by Finnin and Morgan (October 1999).

Other means of topical administration include delivery by electroporation, iontophoresis, phonophoresis, sonophoresis and microneedle or needle-free (*e*.*g*. Powderject™, Bioject™, *etc*.) injection.

Formulations for topical administration may be formulated to be immediate and/or modified release. Modified release formulations include delayed-, sustained-, pulsed-, controlled-, targeted and programmed release.

The compounds of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or hydroxypropylmethylcellulose), blisters and cartridges for use in an inhaler or insufflator may be formulated to contain a powder mix of the compound of the invention, a suitable powder base such as lactose or starch and a performance modifier such as *l*-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1µg to 20mg of the compound of the invention per actuation and the actuation volume may vary from 1µl to 100µl. A typical formulation may comprise a compound of formula (1), propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release using, for example, PGLA. Modified release formulations include delayed-, sustained-, pulsed-, controlled-, targeted and programmed release.

In the case of dry powder inhalers and aerosols, the dosage unit is determined by means of a valve which delivers a metered amount. Units in accordance with the invention are typically arranged to administer a metered dose or "puff" containing from 0.001 mg to 10mg of the compound of formula (1). The overall daily dose will typically be in the range 0.001mg to 40mg which may be administered in a single dose or, more usually, as divided doses throughout the day.

The compounds of formula (1) are particularly suitable for an administration by inhalation

The compounds of the invention may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted and programmed release.

The compounds of the invention may also be administered directly to the eye or ear, typically in the form of drops of a micronised suspension or solution in isotonic, pH-adjusted, sterile saline. Other formulations suitable for ocular and aural administration include ointments, biodegradable (*e.g*. absorbable gel sponges, collagen) and non-biodegradable (*e*.*g*. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

The compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, *i*.*e*. as a carrier, diluent, or solubiliser. Most commonly used for these purposes are alpha-, beta- and gamma-cyclodextrins, examples of which may be found in International Patent Applications Nos. WO 91/11172, WO 94/02518 and WO 98/55148.

Inasmuch as it may desirable to administer a combination of active compounds, for example, for the purpose of treating a particular disease or condition, it is within the scope of the present invention that two or more pharmaceutical compositions, at least one of which contains a compound in accordance with the invention, may conveniently be combined in the form of a kit suitable for coadministration of the compositions.

Thus the kit of the invention comprises two or more separate pharmaceutical compositions, at least one of which contains a compound of formula (1) in accordance with the invention, and means for separately retaining said compositions, such as a container, divided bottle, or divided foil packet. An example of such a kit is the familiar blister pack used for the packaging of tablets, capsules and the like.

The kit of the invention is particularly suitable for administering different dosage forms, for example parenteral, for administering the separate compositions at different dosage intervals, or for titrating the separate compositions against one another. To assist compliance, the kit typically comprises directions for administration and may be provided with a so-called memory aid.

For administration to human patients, the total daily dose of the compounds of the invention is typically in the range 0.001 mg to 5000mg depending, of course, on the mode of administration. For example, an intravenous daily dose may only require from 0.001 mg to 40mg. The total daily dose may be administered in single or divided doses and may, at the physician's discretion, fall outside of the typical range given herein.

These dosages are based on an average human subject having a weight of about 65kg to 70kg. The physician will readily be able to determine doses for subjects whose weight falls outside this range, such as infants and the elderly.

For the avoidance of doubt, references herein to "treatment" include references to curative, palliative and prophylactic treatment.

According to another embodiment of the present invention, the compounds of the formula (1), or pharmaceutically acceptable salts, derived forms or compositions thereof, can also be used as a combination with one or more additional therapeutic agents to be co-administered to a patient to obtain some particularly desired therapeutic end result such as the treatment of pathophysiologically-relevant disease processes including, but not limited to (i) bronchoconstriction, (ii) inflammation, (iii) allergy, (iv) tissue destruction, (v) signs and symptoms such as breathlessness, cough. The second and more additional therapeutic agents may also be a compound of the formula (1), or a pharmaceutically acceptable salt, derived forms or compositions thereof, or one or more β2 agonists known in the art. More typically, the second and more therapeutic agents will be selected from a different class of therapeutic agents.

As used herein, the terms "co-administration", "co-administered" and "in combination with", referring to the compounds of formula (1) and one or more other therapeutic agents, is intended to mean, and does refer to and include the following:
simultaneous administration of such combination of compound(s) of formula (1) and therapeutic agent(s) to a patient in need of treatment, when such components are formulated together into a single dosage form which releases said components at substantially the same time to said patient,
- substantially simultaneous administration of such combination of compound(s) of formula (1) and therapeutic agent(s) to a patient in need of treatment, when such components are formulated apart from each other into separate dosage forms which are taken at substantially the same time by said patient, whereupon said components are released at substantially the same time to said patient,
- sequential administration of such combination compound(s) of formula (1) and therapeutic agent(s) to a patient in need of treatment, when such components are formulated apart from each other into separate dosage forms which are taken at consecutive times by said patient with a significant time interval between each administration, whereupon said components are released at substantially different times to said patient; and
- sequential administration of such combination of compound(s) of formula (1) and therapeutic agent(s) to a patient in need of treatment, when such components are formulated together into a single dosage form which releases said components in a controlled manner whereupon they are concurrently, consecutively, and/or overlapingly administered at the same and/or different times by said patient,
where each part may be administered by either the same or different route.

Suitable examples of other therapeutic agents which may be used in combination with the compound(s) of formula (1), or pharmaceutically acceptable salts, derived forms or compositions thereof, include, but are by no means limited to :
(a) 5-Lipoxygenase (5-LO) inhibitors or 5-lipoxygenase activating protein (FLAP) antagonists,
(b) Leukotriene antagonists (LTRAs) including antagonists of LTB₄, LTC₄, LTD₄, and LTE₄,
(c) Histamine receptor antagonists including H1 and H3 antagonists,
(d) α₁- and α₂-adrenoceptor agonist vasoconstrictor sympathomimetic agents for decongestant use,
(e) muscarinic M3 receptor antagonists or anticholinergic agents,
(f) PDE inhibitors, e.g. PDE3, PDE4 and PDE5 inhibitors,
(g) Theophylline,
(h) Sodium cromoglycate,
(i) COX inhibitors both non-selective and selective COX-1 or COX-2 inhibitors (NSAIDs),
(j) Oral and inhaled glucocorticosteroids,
(k) Monoclonal antibodies active against endogenous inflammatory entities,
(I) Anti-tumor necrosis factor (anti-TNF-α) agents,
(m)Adhesion molecule inhibitors including VLA-4 antagonists,
(n) Kinin-B₁ - and B₂ -receptor antagonists,
(o) Immunosuppressive agents,
(p) Inhibitors of matrix metalloproteases (MMPs),
(q) Tachykinin NK₁, NK₂ and NK₃ receptor antagonists,
(r) Elastase inhibitors,
(s) Adenosine A2a receptor agonists,
(t) Inhibitors of urokinase,
(u) Compounds that act on dopamine receptors, e.g. D2 agonists,
(v) Modulators of the NFκβ pathway, e.g. IKK inhibitors,
(w) modulators of cytokine signalling pathyways such as p38 MAP kinase or syk kinase,
(x) Agents that can be classed as mucolytics or anti-tussive, and
(y) Antibiotics.

According to the present invention, combination of the compounds of formula (1) with :
- H3 antagonists,
- Muscarinic M3 receptor antagonists,
- PDE4 inhibitors,
- glucocorticosteroids,
- Adenosine A2a receptor agonists,
- Modulators of cytokine signalling pathyways such as p38 MAP kinase or syk kinase, or,
- Leukotriene antagonists (LTRAs) including antagonists of LTB₄, LTC₄, LTD₄, and LTE₄,
are preferred.

According to the present invention, combination of the compounds of formula (1) with :
- glucocorticosteroids, in particular inhaled glucocorticosteroids with reduced systemic side effects, including prednisone, prednisolone, flunisolide, triamcinolone acetonide, beclomethasone dipropionate, budesonide, fluticasone propionate, ciclesonide, and mometasone furoate, or
- muscarinic M3 receptor antagonists or anticholinergic agents including in particular ipratropium salts, namely bromide, tiotropium salts, namely bromide, oxitropium salts, namely bromide, perenzepine, and telenzepine,
are more preferred.

It is to be appreciated that all references herein to treatment include curative, palliative and prophylactic treatment. The description, which follows, concerns the therapeutic applications to which the compounds of formula (1) may be put.

The compounds of formula (1) have the ability to interact with the β2 receptor and thereby have a wide range of therapeutic applications, as described further below, because of the essential role which the β2 receptor plays in the physiology of all mammals.

Therefore, a further aspect of the present invention relates to the compounds of formula (1), or pharmaceutically acceptable salts, derived forms or compositions thereof, for use in the treatment of diseases, disorders, and conditions in which the β2 receptor is involved. More specifically, the present invention also concerns the compounds of formula (1), or pharmaceutically acceptable salts, derived forms or compositions thereof, for use in the treatment of diseases, disorders, and conditions selected from the group consisting of :
- asthma of whatever type, etiology, or pathogenesis, in particular asthma that is a member selected from the group consisting of atopic asthma, non-atopic asthma, allergic asthma, atopic bronchial IgE-mediated asthma, bronchial asthma, essential asthma, true asthma, intrinsic asthma caused by pathophysiologic disturbances, extrinsic asthma caused by environmental factors, essential asthma of unknown or inapparent cause, non-atopic asthma, bronchitic asthma, emphysematous asthma, exercise-induced asthma, allergen induced asthma, cold air induced asthma, occupational asthma, infective asthma caused by bacterial, fungal, protozoal, or viral infection, non-allergic asthma, incipient asthma, wheezy infant syndrome and bronchiolytis,
- chronic or acute bronchoconstriction, chronic bronchitis, small airways obstruction, and emphysema,
- obstructive or inflammatory airways diseases of whatever type, etiology, or pathogenesis, in particular an obstructive or inflammatory airways disease that is a member selected from the group consisting of chronic eosinophilic pneumonia, chronic obstructive pulmonary disease (COPD), COPD that includes chronic bronchitis, pulmonary emphysema or dyspnea associated or not associated with COPD, COPD that is characterized by irreversible, progressive airways obstruction, adult respiratory distress syndrome (ARDS), exacerbation of airways hyper-reactivity consequent to other drug therapy and airways disease that is associated with pulmonary hypertension,
- bronchitis of whatever type, etiology, or pathogenesis, in particular bronchitis that is a member selected from the group consisting of acute bronchitis, acute laryngotracheal bronchitis, arachidic bronchitis, catarrhal bronchitis, croupus bronchitis, dry bronchitis, infectious asthmatic bronchitis, productive bronchitis, staphylococcus or streptococcal bronchitis and vesicular bronchitis,
- acute lung injury,
- bronchiectasis of whatever type, etiology, or pathogenesis, in particular bronchiectasis that is a member selected from the group consisting of cylindric bronchiectasis, sacculated bronchiectasis, fusiform bronchiectasis, capillary bronchiectasis, cystic bronchiectasis, dry bronchiectasis and follicular bronchiectasis.

A still further aspect of the present invention also relates to the use of the compounds of formula (1), or pharmaceutically acceptable salts, derived forms or compositions thereof, for the manufacture of a drug having a β2 agonist activity. In particular, the present inventions concerns the use of the compounds of formula (1), or pharmaceutically acceptable salts, derived forms or compositions thereof, for the manufacture of a drug for the treatment of β2-mediated diseases and/or conditions, in particular the diseases and/or conditions listed above.

As a consequence, the present invention provides a particularly interesting method to treat a mammal, including a human being, with an effective amount of a compound of formula (1), or a pharmaceutically acceptable salt, derived form or composition thereof. More precisely, the present invention provides a particularly interesting method for the treatment of a β2-mediated diseases and/or conditions in a mammal, including a human being, in particular the diseases and/or conditions listed above, comprising admidministering said mammal with an effective amount of a compound of formula (1), its pharmaceutically acceptable salts and/or derived forms.

The following examples illustrate the preparation of the compounds of the formula (1):

### Preparation 1

### 8-(Benzyloxy)-5-(bromoacetyl)quinolin-2(1H)-one

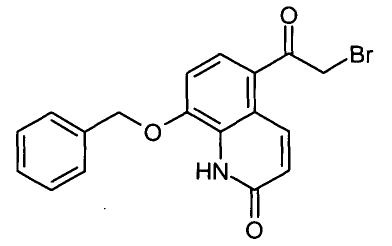

A solution of tetra-n-butylammonium tribromide (109g, 0.226mol) in tetrahydrofuran (250ml) was added dropwise over 90 minutes to a stirred suspension of 8-(benzyloxy)-5-(1-oxoethyl)-1H-quinol-2-one (WO 2003/042164) (38.9g, 0.133mol) in a mixture of tetrahydrofuran (470ml) and methanol (325ml) at room temperature. The resulting mixture was stirred for 17 hours, concentrated under reduced pressure at 30°C and the brown solid residue was suspended in methanol (390ml). Water (780ml) was added slowly with cooling using a cold water bath. The resulting brown suspension was stirred for 30 minutes and filtered, washing with water (600ml), and the solid was dried by suction overnight.
The crude product (89.2g) was triturated with a mixture of dichloromethane and methanol (1:1, 2 ml/g) for 90 minutes. The solid was filtered and washed with dichloromethane (53ml). The solid was then heated in a mixture of dichloromethane (39ml) and methanol (8ml) for 2 hours under reflux, cooled to room temperature and stirred for 1 hour. Filtration of the solid followed by washing with dichloromethane:methanol (9:1, 30ml) afforded the title compound as a pale brown solid, 37.15 g.
¹H nmr (CDCl₃, 400MHz) δ: 4.42 (s, 2H), 5.25 (s, 2H), 6.75 (d, 1H), 7.03 (d, 1H), 7.40 (s, 5H), 7.67 (d, 1H), 8.78 (d, 1H), 9.23 (br s, 1H).
LRMS : m/z ES⁺ 372, 374 [MH⁺]

### Preparation 2

### 8-(Benzyloxy)-5-[(1R)-2-bromo-1-hydroxyethyl]quinolin-2(1H)-one

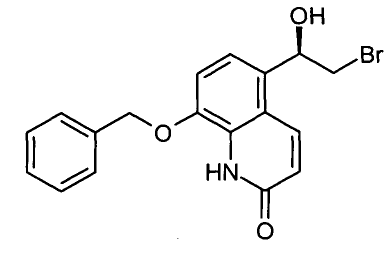

A mixture of trimethylboroxine (0.74ml 5.34mmol) and (*R*)-2-(diphenylhydroxymethyl)pyrrolidine (2.03g, 8.0mmol) in toluene (13.6ml) was stirred for 40 minutes. Toluene (13.6ml) was added and the mixture was concentrated to 8 ml by distillation at 1 atm. Another portion of toluene (13.6 ml) was added and the mixture was concentrated to 8 ml by distillation at 1 atm. This procedure was repeated another three times using anhydrous toluene.
A suspension of the compound from preparation 1 (12.0g, 32.2mmol) in anhydrous tetrahydrofuran (180ml) under nitrogen was cooled to -5°C using an ice-isopropanol bath. The toluene solution of the oxoborolidine catalyst prepared above was added in one portion via syringe. Borane dimethyl sulphide complex (5.0 ml, 50mmol) was dissolved in anhydrous tetrahydrofuran (50ml) under nitrogen. A portion of this (40ml, 40mmol) was added at -6°C via syringe using a syringe pump over 3.5 hours. Methanol (40ml) was added slowly keeping the reaction mixture between -5°C and 0°C. The mixture was stirred for 30 minutes and allowed to warm to room temperature. The solvent was removed under reduced pressure and the residue was slurried in methanol (200ml) and concentrated to dryness (twice).
The crude product was dissolved in dichloromethane (1300ml) containing a little methanol, and washed with hydrochloric acid (1M, 240ml) and water (2 × 300ml). The organic solution was dried (MgSO₄) and concentrated under reduced pressure and the product recrystallised from glacial acetic acid to give the desired product as an off-white solid, 11.0g.
¹H nmr (CDCl₃, 400MHz) δ: 2.88 (brd, 1H,), 3.58 (dd, 1H), 3.62 (dd, 1H), 5.16 (s, 2H), 5.29 (m, 1H), 6.68 (d, 1H), 7.01 (d, 1H), 7.25 (d, 1H), 7.39 (s, 5H), 8.04 (d, 1H), 9.18 (br s, 1H).
LRMS :m/z ES⁺ 374, 376 [MH⁺]

### Preparation 3

### 8-(Benzyloxy)-5-((1R)-2-bromo-1-{[tert-butyl(dimethyl)silyl]oxy}ethyl)quinolin-2(1H)-one

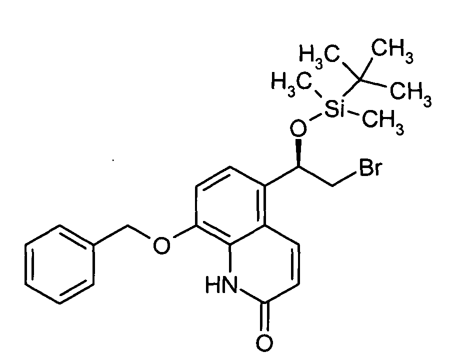

*tert*-Butyldimethylsilyl triflate (21.4ml, 93.15mmol) was added dropwise over 10 minutes to a stirred suspension of the compound from preparation 2 (17.42g, 46.6mmol) and 2,6-lutidine (10.9ml, 93.15mmol) in anhydrous dichloromethane (460ml) under nitrogen at 4°C. The mixture was allowed to warm to room temperature and stirred for 18 hours. The solution was washed with hydrochloric acid (1M, 2 × 150ml), water (2 × 200ml), dried (MgSO₄) and concentrated under reduced pressure. The residue was azeotroped twice with cyclohexane (300ml) to give an orange gum (27.2 g). The crude product was purified by column chromatography on silica gel, eluting with dichloromethane:ethyl acetate (90:10). The product was recrystallised from cyclohexane to give the title compound as a colourless solid, 18.4g.
¹H nmr (CDCl₃, 400MHz) δ: -0.15 (s, 3H), 0.10 (s, 3H), 0.85 (s, 9H), 3.46 (dd, 1H), 3.56 (dd, 1H), 5.14 (s, 2H), 5.15 (dd, 1H), 6.67 (d, 1H), 7.00 (d, 1H), 7.14 (d, 1H), 7.40 (s, 5H), 8.20 (d, 1H), 9.17 (br s, 1H).
LRMS :m/z ES⁺ 488, 490 [MH⁺].

### Preparation 4

### (3-Bromo-phenyl)-acetic acid methyl ester

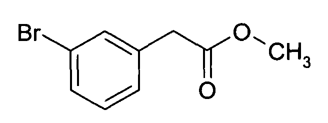

Acetyl chloride (0.7ml, 9.3mmol) was slowly added to a solution of (3-bromophenyl)-acetic acid (20.0g, 93mmol) in methanol (500ml) at 0°C under nitrogen and the reaction was allowed to warm gradually to room temperature over a period of 5 hours. The solvent was removed under reduced pressure and the residue dissolved in dichloromethane, dried (Na₂SO₄) and concentrated under reduced pressure to give the title compound as a colourless oil, 20.6g.
¹H nmr (CDCl₃, 400MHz) δ: 3.59 (s, 2H), 3.70 (s, 3H), 7.17-7.24 (m, 2H), 7.37-7.45 (m, 2H)
LRMS :m/z ES⁺ 253 [M+Na]⁺.

### Preparation 5

### (4-Bromo-phenyl)-acetic acid methyl ester

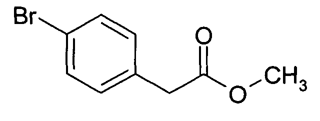

Hydrogen chloride was bubbled through an ice-cold solution of (4-bromophenyl)-acetic acid (48.1 g, 224mmol) in methanol (600ml) and once saturated the solution was allowed to warm to room temperature. The reaction was concentrated under reduced pressure and the residue basified with 1N sodium hydroxide solution and extracted with ethyl acetate (2x200ml). The combined organic extracts were washed with brine (200ml), dried (MgSO₄) and evaporated under reduced pressure to afford the title compound as a clear oil, 50.1g.
¹H nmr (CDCl₃, 400MHz) δ: 3.58 (s, 2H), 3.69 (s, 3H), 7.15 (d, 2H), 7.44 (d, 2H)
LRMS : m/z ES⁺ 253, 255 [M+Na]⁺

### Preparation 6

### [3-(2-Oxo-propyl)-phenyl]-acetic acid methyl ester

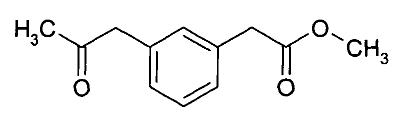

A solution of the bromide from preparation 4 (15.0g, 65.0mmol), tributyltin methoxide (28.3ml, 98.0mmol), isopropenyl acetate (10.8ml, 98.0mmol), palladium(II) acetate (750mg, 3.30mmol) and tri-*ortho*-tolylphosphine (2.0g, 6.5 mmol) were stirred together in toluene (75ml) at 100°C under nitrogen for 5 hours. After cooling the reaction was diluted with ethyl acetate (150ml) and 4M aqueous potassium fluoride solution (90ml) and stirred for 15 minutes. The mixture was filtered through Arbocel® and the organic phase separated and reduced under reduced pressure. The residue was purified by column chromatography on silica gel eluting with a solvent gradient of diethyl ether:pentane:dichloromethane (0:100:0 to 25:75:0 to 0:0:100, by volume) to give the title compound as a pale yellow oil, 12.6g.
¹H nmr (CDCl₃, 400MHz) δ : 2.15 (s, 3H), 3.61 (s, 2H), 3.69 (m, 5H), 7.10-7.13 (m, 2H), 7.19 (d, 1H), 7.30 (dd, 1H).
LRMS : m/z ES⁺ 224 [M+NH₄]⁺

### Preparation 7

### [4-(2-Oxo-propyl)-phenyl]-acetic acid methyl ester

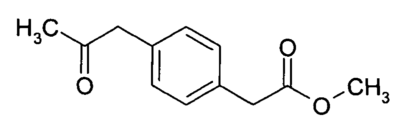

The title compound was obtained as a clear oil in 84% yield from the compound from preparation 5, following the procedure described in preparation 6.
¹H nmr (CDCl₃, 400MHz) δ: 2.15 (s, 3H), 3.60 (s, 2H), 3.68 (m, 5H), 7.16 (d, 2H), 7.25 (d, 2H).
LRMS : m/z ES⁺ 224 [M+NH₄]⁺

### Preparation 8

### Diethyl 2,2'-(1,3-phenylene)diacetate

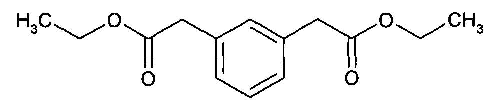

2,2'-(1,3-Phenylene)diacetic acid (10.0g, 51mmol) was dissolved in ethanol (100ml) and the solution treated dropwise with catalytic acetyl chloride (2.5ml). The reaction mixture was stirred at reflux for 18 hours before being allowed to cool and concentrated under reduced pressure. The residue was taken up in ethyl acetate (100ml) and washed with sodium bicarbonate solution (3x50ml) and brine (3x50ml). The organic phase was dried (MgSO₄) and concentrated under reduced pressure. The residue was triturated with pentane to yield the title product, 11.8g.
¹Hnmr (CDCl₃, 400MHz) δ: 1.31 (t, 6H), 3.65 (s, 4H), 4.20 (q, 4H), 7.24-7.36 (m, 4H).
LRMS : m/z ES⁺ 251 [MH]⁺

### Preparation 9

### [3-(2-Ethoxy-2-oxoethyl)phenyl]acetic acid

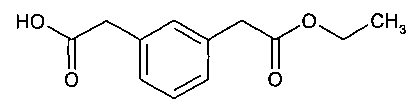

A solution of the diester from preparation 8 (44.3g, 177mmol) and 2,2'-(1,3-phenylene)diacetic acid (59.2g, 308mmol) in ethanol (24ml) and dioxan (290ml) was treated dropwise with 12M hydrochloric acid (4.9ml, 58.8mmol). The reaction mixture was stirred at reflux for 18 hours before being allowed to cool and concentrated to low volume. The reaction mixture was diluted with toluene (125ml) and the resulting slurry filtered. The filtrate was concentrated under reduced pressure and the residue taken up in water and neutralised with sodium bicarbonate. The mixture was diluted with ethyl acetate (200ml) and the organic layer was separated and washed with sodium bicarbonate solution (5x30ml) and brine (50ml). The combined aqueous extracts were acidified to pH 3 with 6M hydrochloric acid and extracted with ether (3x30ml). The organics were combined, dried (MgSO₄) and concentrated under reduced pressure. The residue was triturated with pentane giving the title compound as a colourless solid 10.8g.
¹Hnmr (CD₃OD, 400MHz) δ: 1.25 (t, 3H), 3.60 (m, 2H), 3.63 (m, 2H), 4.15 (q, 2H), 7.18-7.32 (m, 4H)
LRMS : m/z ES⁺ 245 [MNa]⁺

### Preparation 10

### [3-(2-Hydroxy-2-methyl-propyl)-phenyl]-acetic acid

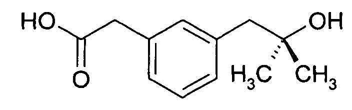

A solution of the acid of preparation 9 (6.85g, 32mmol) in diethyl-ether (100ml) was cooled to 0°C and treated with a 3M solution of methylmagnesium bromide in ether (23.5ml, 70.0mmol). The reaction mixture was allowed to warm gradually to room temperature. After 2 hours the reaction was quenched by addition of saturated aqueous ammonium chloride solution (200ml). The organic phase was separated and washed with brine (100ml), dried (MgSO₄) and concentrated under reduced pressure. Purification by column chromatography on silica gel eluting with pentane:dichloromethane 60:40 to 0:100 gave the title compound as a colourless oil, 6.23g.
¹H nmr (CDCl₃, 400MHz) δ: 1.22 (s, 6H), 2.75 (s, 2H), 3.63 (s, 2H), 7.12-7.30 (m, 4H).
LRMS : m/z ES⁺ 209 [MH]⁺

### Preparation 11

### {3-[2-(2-Chloro-acetylamino)-2-methyl-propyl]-phenyl}-acetic acid

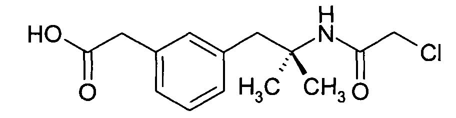

2-Chloroacetonitrile (8.8ml, 140mmol) was added to a solution of the alcohol from preparation 10 (16.0g, 70mmol), in acetic acid (33ml). The resulting solution was cooled to 0°C, treated with concentrated sulphuric acid (33ml), and the reaction mixture allowed to warm gradually to room temperature. After 4 hours the reaction mixture was poured onto ice and basified with solid sodium carbonate. The solution was extracted with ethyl acetate (2x500ml) and the combined organic extracts dried (MgSO₄) and concentrated under reduced pressure to give the title product as a colourless solid, 19.0g.
¹H nmr (CDCl₃, 400MHz) δ : 1.36 (s, 6H), 3.02 (s, 2H), 3.62 (s, 2H), 3.95 (s, 2H), 6.19 (br s, 1H), 7.06-7.31 (m, 4H)
LRMS : m/z ES⁻ 282, 284 [M-H]⁻

### Preparation 12

### {3-[(2R)-2-((1R)-1-Phenyl-ethylamino)-propyl]-phenyl}-acetic acid methyl ester hydrochloride

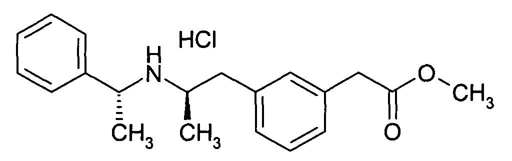

A solution of the ketone from preparation 6 (8.5g, 41.2mmol), (*R*)-α-methyl benzylamine (4.8ml, 37.2mmol), sodium triacetoxyborohydride (11.6g, 56mmol) and acetic acid (2.2ml, 38mmol) in dichloromethane (400ml) was stirred at room temperature for 48 hours. The reaction mixture was quenched by addition of saturated aqueous sodium bicarbonate (200ml) and allowed to stir until effervescence ceased. The organic phase was separated and the aqueous phase extracted with dichloromethane (100ml). The combined organic solutions were dried (MgSO₄) and concentrated under reduced pressure. Purification by column chromatography on silica gel eluting with dichloromethane:methanol:0.88 ammonia (99:1:0.1 to 95:5:0.5 by volume) gave a 4:1 mixture of diastereomers (*R,R* major) as a pale yellow oil (8.71g). Treatment with excess 1M hydrogen chloride in methanol followed by three successive crystallisations (diisopropylether/methanol) gave the title compound as a colourless crystalline solid, 5.68g.
¹H nmr (CD₃OD, 400MHz) δ: 1.18 (d, 3H), 1.68 (d, 3H), 2.60-2.66 (m, 1H), 3.15-3.26 (m, 1H), 3.25-3.30 (m, 1H), 3.30 (s, 3H), 3.62 (s, 2H), 4.59 (q, 1H), 7.00 (m, 2H), 7.17 (m, 1H), 7.27 (m, 1H), 7.50 (m, 5H).
LRMS : m/z ES⁺ 312 [MH]⁺

### Preparation 13

### {4-[(2R)-2-((1R)-1-Phenyl-ethylamino)-propyl]-phenyl}-acetic acid methyl ester hydrochloride

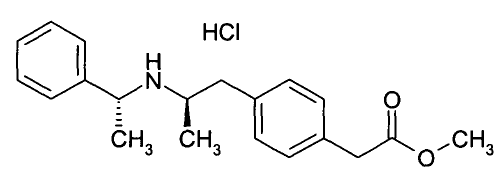

A mixture of the ketone from preparation 7 (31.9g, 155mmol), (*R*)-α-methyl benzylamine (18.74g, 155mmol), acetic acid (6.92g, 155mmol) and sodium triacetoxyborohydride (49.2g, 232mmol) in dichloromethane (500ml) was stirred at room temperature for 7 hours. The mixture was diluted with dichloromethane (500ml), basified to pH 12 using 1N sodium hydroxide, washed with brine (500ml) and dried (MgSO₄) and evaporated under reduced pressure. The yellow residue was dissolved in ethyl acetate (200ml), acidified with saturated methanolic hydrogen chloride and the mixture evaporated under reduced pressure. The product was recrystallised from methanol:diisopropyl ether to afford the title compound as white crystals, 21.7g.
¹H nmr (CD₃OD, 400MHz) δ: 1.18 (d, 3H), 1.70 (d, 3H), 2.62 (dd, 1H), 3.18 (m, 1H), 3.30 (m, 1H), 3.62 (s, 2H), 3.66 (s, 3H), 4.62 (q, 1H), 7.06 (d, 2H), 7.21 (d, 2H), 7.53 (m, 5H).
LRMS : m/z ES⁺ 312 [MH]⁺

### Preparation 14

### Methyl {3-[(2R)-2-aminopropyl]phenyl}acetate

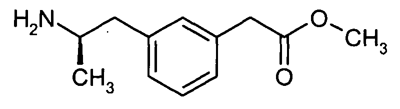

A solution of the amine from preparation 12 (7.69g, 22mmol) and ammonium formate (6.94g, 110mmol) in ethanol (100ml) was heated to 75°C in the presence of 20% of palladium hydroxide-on-charcoal (2.00g). After 90 minutes the reaction mixture was cooled to room temperature, filtered through Arbocel® and the filtrate concentrated under reduced pressure. The residue was partitioned between dichloromethane (100ml) and 0.88 ammonia (100ml) and the organic phase separated. The aqueous phase was extracted with dichloromethane (100ml) and the combined organic extracts dried (MgSO₄) and concentrated under reduced pressure to give the title compound as a colourless oil, 4.78g.
¹H nmr (CD₃OD, 400MHz) δ :1.06 (d, 3H), 2.57-2.67 (m, 2H), 3.05-3.12 (m, 1H), 3.63 (s, 2H), 3.67 (s, 3H), 7.09-7.13 (m, 3H), 7.25 (m, 1H).
LRMS : m/z ES⁺ 208 [MH]⁺

### Preparation 15

### Methyl {3-[(2R)-2-aminopropyl]phenyl}acetate

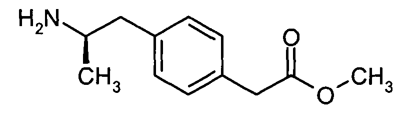

The title compound was prepared by a method similar to that described for preparation 14 using the compound from preparation 13.
¹Hnmr (CDCl₃, 400MHz) δ: 1.14 (d, 3H), 2.79 (m, 2H), 3.39 (m, 1H), 3.60 (s, 2H), 3.68 (s, 3H), 7.17 (d, 2H), 7.22 (d, 2H).
LRMS : m/z ES⁺ 208 [MH]⁺

### Preparation 16

### Methyl [3-(2-amino-2-methylpropyl)phenyl]acetate

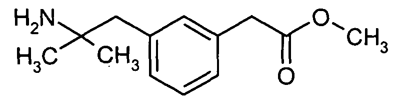

A solution of the amide from preparation 11 (5.1g, 18mmol), thiourea (1.6g, 21 mmol) and acetic acid (18ml) in ethanol (80ml) was heated to reflux under a nitrogen atmosphere for 16 hours. The reaction mixture was cooled and filtered. The filtrate was concentrated under reduced pressure the residue dissolved in ethanol (150ml), the solution saturated with hydrogen chloride gas and heated to reflux for 16 hours. The solvent was reduced *in vacuo* and the residue partitioned between ethyl acetate (200ml) and 5% aqueous sodium carbonate (200ml). The organic extract was washed with saturated sodium chloride (100ml), dried (Na₂SO₄) and reduced *in vacuo.* The residue was purified by strong cation exchange resin, eluting with methanol and then 2N ammonia in methanol to elute the product. The eluant was concentrated *in vacuo* to give the title compound as a yellow oil, 2.68g.
¹H nmr (CDCl₃, 400MHz) δ :1.14 (s, 6H), 2.68 (s, 2H), 3.62 (s, 2H), 3.69 (s, 3H), 7.08-7.16 (m, 3H), 7.23-7.27 (m, 1H).
LRMS : m/z ES⁺ 236 [MH]⁺

### Preparation 17

### Methyl (4-{(2R)-2-[((2R)-2-[8-(benzyloxy)-2-oxo-1,2-dihydroquinolin-5-yl]-2-{[tert-butyl(dimethyl)silyl]oxy}ethyl)amino]propyl}phenyl)acetate

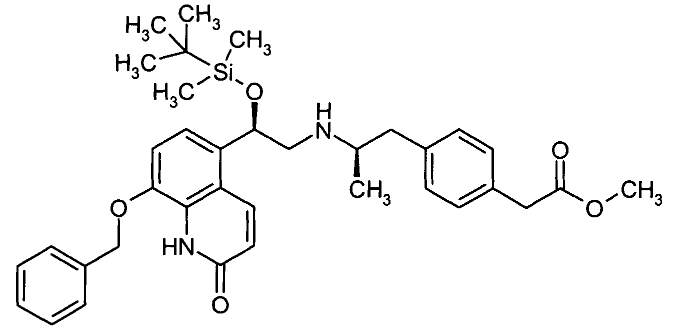

A mixture of the bromide from preparation 3 (3g, 6.14mmol) and the amine from preparation 15 (2.55g, 12mmol) were melted at 90°C for 18 hours. The cooled residue was dissolved in dichloromethane and the solution purified by column chromatography on silica gel using dichloromethane:methanol:0.88 ammonia (99.8: 0:0.2 to 94.8:5:0.2) to give a gum. This was dissolved in ether and the solution evaporated under reduced pressure to afford the title compound as a white foam, 1.5g. The bromide and amine starting materials were collected from the column, the fractions evaporated under reduced pressure and then combined and heated at 90°C for a further 72 hours. The cooled residue was dissolved in methanol (40ml) and purified by column chromatography using a SCX Isolute® ion-exchange column and methanol:1M methanolic ammonia (100:0 to 0:100) as eluant to give additional title compound, 2.5g (in total).
¹H nmr (CDCl₃, 400MHz) δ: -0.22 (s, 3H), 0.02 (s, 3H), 0.82 (s, 9H), 1.01 (d, 3H), 2.25 (m, 1H), 2.66-2.80 (m, 2H), 2.87 (m, 1H), 2.97 (m, 1H), 3.58 (s, 2H), 3.67 (s, 3H), 5.08 (m, 1H), 5.19 (s, 2H), 6.65 (d, 1H), 6.98-7.17 (m, 6H), 7.43 (m, 5H), 8.25 (d, 1H), 9.20 (s, 1H).
LRMS : m/z APCI⁺ 615 [MH]⁺

### Preparation 18

### Methyl (3-{(2R)-2-[((2R)-2-[8-(benzyloxy)-2-oxo-1,2-dihydroquinolin-5-yl]-2-{[tert-butyl(dimethyl)silyl]oxy}ethyl)amino]propyl}phenyl)acetate

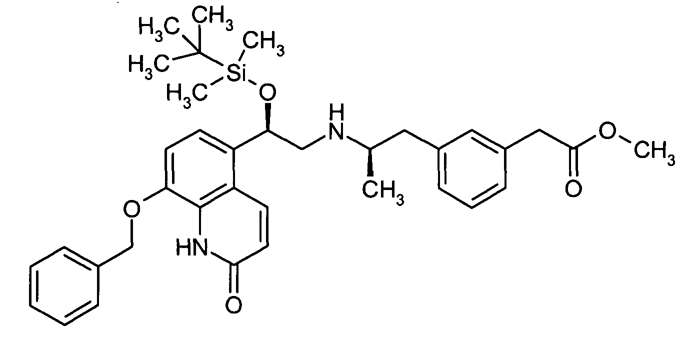

The bromide from preparation 3 (3.6g, 7.37mmol), N-ethyldiisopropylamine (1.3g, 7.37mmol), dimethylsulphoxide (1.4ml) and dichloromethane (30ml) were added to the amine from preparation 14 (1.53g, 7.37mmol) and the reaction mixture heated at 90°C for 18 hours, allowing dichloromethane to evaporate. The cooled residue was purified by column chromatography using a SCX Isolute® ion exchange cartridge, using methanol and then 1M methanolic ammonia as eluant. The orange oil was purified by column chromatography on silica gel using an elution gradient of dichloromethane:methanol:0.88 ammonia (100:0:0 to 98:2:0.2) to afford the title compound as an orange oil, 2.17g.
¹H nmr (CD₃OD, 400MHz) δ: -0.24 (s, 3H), 0.02 (s, 3H), 0.82 (s, 9H), 1.06 (d, 3H), 2.50-2.62 (m, 2H), 2.74 (m, 1H), 2.86 (m, 1H), 3.04 (m, 1H), 3.53 (s, 2H), 3.64 (s, 3H), 5.13 (m, 1H), 5.32 (s, 2H), 6.62 (d, 1H), 6.88 (d, 1H), 6.96 (m, 1H), 7.02-7.13 (m, 4H), 7.31-7.41 (m, 3H), 7.55 (d, 2H), 8.40 (d, 1H).
LRMS : m/z ES⁺ 637 [MNa]⁺

### Preparation 19

### Methyl (3-{2-[((2R)-2-[8-(benzyloxy)-2-oxo-1,2-dihydroquinolin-5-yl]-2-{[tert-butyl(dimethyl)silyl]oxy}ethyl)amino]-2-methylpropyl}phenyl)acetate

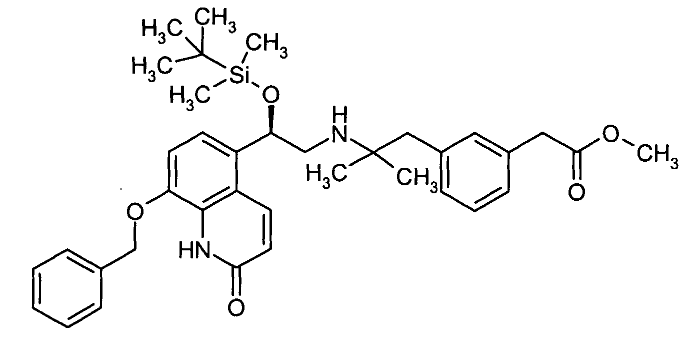

A mixture of the bromide from preparation 3 (5.0g, 10mmol), and the amine from preparation 16 (4.81g, 20mmol) in dichloromethane (50ml) was heated at 90°C for 48 hours, allowing the solvent to evaporate off to give a melt. The cooled mixture was passed down a SCX Isolute® ion-exchange cartridge, washing through with methanol, then 1M methanolic ammonia. The product containing washings were evaporated under reduced pressure and the residue purified by column chromatography on silica gel using dichloromethane:methanol:0.88 ammonia (98:2:0.2 to 97:3:0.3) to afford the title compound as an orange gum, 3.11g.
¹H nmr (CD₃OD, 400MHz) δ: -0.25 (s, 3H), 0.01 (s, 3H), 0.79 (s, 9H), 1.03 (s, 3H), 1.06 (s, 3H), 2.62-2.71 (m, 2H), 2.74 (dd, 1H), 2.97 (dd, 1H), 3.59 (s, 2H), 3.66 (s, 3H), 5.14 (m, 1H), 5.33 (s, 2H), 6.68 (d, 1H), 7.00 (d, 1H), 7.07 (s, 1H), 7.10-7.18 (m, 4H), 7.31-7.41 (m, 3H), 7.52 (d, 2H), 8.48 (d, 1H).
LRMS : m/z ES⁺ 629 [MH]⁺

### Preparation 20

### Methyl [4-((2R)-2-{[(2R)-2-{[tert-butyl(dimethyl)silyl]oxy}-2-(8-hydroxy-2-oxo-1,2-dihydroquinolin-5-yl)ethyl]amino}propyl)phenyl]acetate

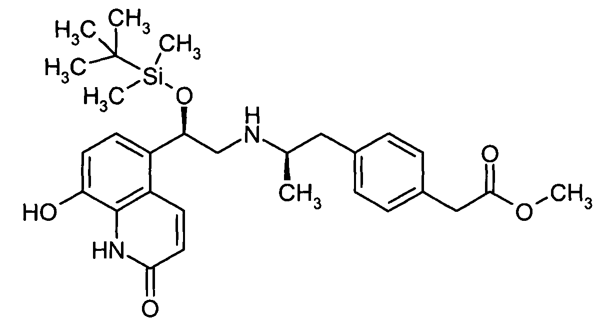

A mixture of the compound from preparation 17 (2.5g, 4mmol) and 10% palladium on charcoal (350mg) in methanol (40ml) was hydrogenated at 60 psi and room temperature for 40 hours. The mixture was filtered through Arbocel® and the filtrate evaporated under reduced pressure. The residue was azeotroped with ether to afford the title compound as a foam, 2.1g.
¹H nmr (CDCl₃, 400MHz) δ: -0.18 (s, 3H), 0.02 (s, 3H), 0.82 (s, 9H), 1.12 (d, 3H), 2.62 (m, 2H), 2.80 (m, 1H), 2.90 (m, 1H), 3.18 (m, 1H), 3.62 (s, 2H), 3.75 (s, 3H), 5.08 (m, 1H), 6.65 (m, 2H), 6.83 (m, 1H), 6.98 (d, 2H), 7.10 (d, 2H), 8.24 (d, 1H).
HRMS : 525.2790 [MH]⁺ [C₂₉H₄₀N₂O₅Si] requires 525.2779

### Preparation 21

### Methyl [3-((2R)-2-{[(2R)-2-{[tert-butyl(dimethyl)silyl]oxy}-2-(8-hydroxy-2-oxo-1,2-dihydroquinolin-5-yl)ethyl]amino}propyl)phenyl]acetate

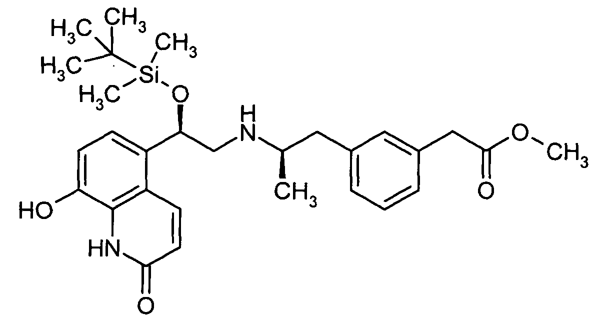

A mixture of the compound from preparation 18 (2.49g, 4.75mmol), ammonium formate (1.09g, 23.7mmol) and 20% palladium on charcoal (250mg) in ethanol (25ml) was heated at 80°C for 2 hours. Tlc analysis showed starting material remaining so additional ammonium formate (200mg, 4.75mmol) was added and the reaction stirred for a further hour. The cooled mixture was filtered through Arbocel®, washing through with ethanol and the filtrate evaporated under reduced pressure. The residual oil was purified by column chromatography on silica gel using an elution gradient of dichloromethane:methanol:0.88 ammonia (98:2:0.2 to 95:5:0.5) to afford the title compound as a yellow foam, 1.31g.
¹H nmr (CD₃OD, 400MHz) δ: -0.24 (s, 3H), 0.02 (s, 3H), 0.82 (s, 9H), 1.07 (d, 3H), 2.52-2.64 (m, 2H), 2.74 (m, 1H), 2.88 (m, 1H), 3.04 (dd, 1H), 3.56 (s, 2H), 3.64 (s, 3H), 5.10 (m, 1H), 6.60 (d, 1H), 6.90 (m, 2H), 6.98 (m, 2H), 7.06 (m, 2H), 8.40 (d, 1H).
LRMS : m/z ES⁺ 525 [MH]⁺

### Preparation 22

### Methyl [3-(2-{[(2R)-2-{[tert-butyl(dimethyl)silyl]oxy}-2-(8-hydroxy-2-oxo-1,2-dihydroquinolin-5-yl)ethyl]amino}-2-methylpropyl)phenyl]acetate

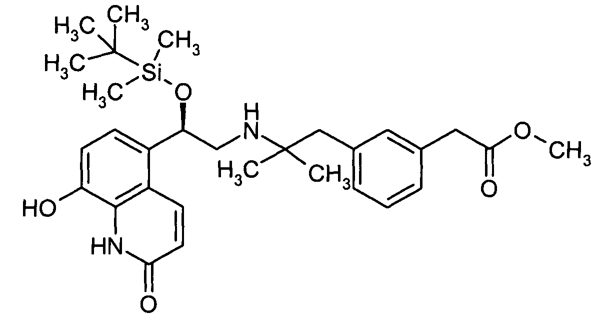

A mixture of the compound from preparation 19 (3.04g, 4.83mmol) and 10% palladium on charcoal (300mg) in ethanol (50ml) was hydrogenated at 60psi and room temperature for 42 hours. The reaction mixture was filtered through Arbocel®, washing through with ethanol. The filtrate was evaporated under reduced pressure and the residual gum was purified by column chromatography on silica gel using dichloromethane:methanol:0.88 ammonia (97:3:0.3 to 95:5:0.5) as elution gradient to afford the title compound as a yellow foam, 1.88g.
¹H nmr (CD₃OD, 400MHz) δ: -0.24 (s, 3H), 0.01 (s, 3H), 0.79 (s, 9H), 1.04 (s, 3H), 1.08 (s, 3H), 2.63-2.73 (m, 2H), 2.76 (dd, 1H), 3.00 (dd, 1H), 3.60 (s, 2H), 3.66 (s, 3H), 5.13 (m, 1H), 6.65 (d, 1H), 6.95 (d, 1H), 7.01 (d, 1H), 7.07-7.20 (m, 4H), 8.46 (d, 1H).
LRMS : m/z ES⁺ 539 [MH]⁺

### Preparation 23

### [4-((2R)-2-{[(2R)-2-{[tert-Butyl(dimethyl)silyl]oxy}-2-(8-hydroxy-2-oxo-1,2-dihydroquinolin-5-yl)ethyl]amino}propyl)phenyl]acetic acid

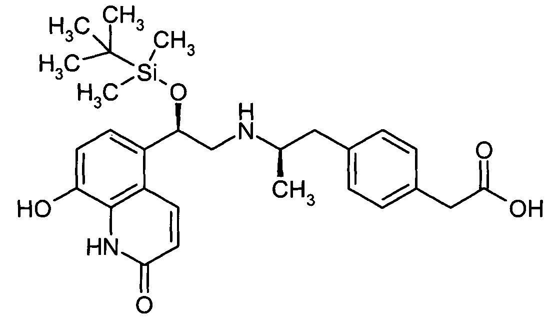

Sodium hydroxide (4.0ml, 5M, 20mmol) was added dropwise to a solution of the compound from preparation 20 (2.1g, 4.0mol) in water (3ml) and dioxan (24ml), and the reaction stirred at room temperature for 18 hours. The reaction mixture was concentrated under reduced pressure, the residue dissolved in water and extracted with ether (3x20ml). The aqueous phase was treated with hydrochloric acid (2M, 10.16ml, 20.3mmol) and the resulting precipitate filtered off and dried *in vacuo* to afford the title compound as a pink powder, 1.6g.
¹H nmr (CD₃OD, 400MHz) δ: -0.18 (s, 3H), 0.10 (s, 3H), 0.82 (s, 9H), 1.12 (d, 3H), 2.78 (m, 1H), 2.92 (m, 1H), 3.12 (m, 1H), 3.35 (m, 2H), 3.58 (s, 2H), 5.40 (m, 1H), 6.65 (d, 1H), 6.98 (m, 1H), 7.10 (m, 3H), 7.22 (m, 2H), 8.34 (d, 1H).
HRMS : 511.2630 [MH]⁺ [C₂₈H₃₈N₂O₅Si] requires 511.2623

### Preparation 24

### [3-((2R)-2-{[(2R)-2-{[tert-Butyl(dimethyl)silyl]oxy}-2-(8-hydroxy-2-oxo-1,2-dihydroquinolin-5-yl)ethyl]amino}propyl)phenyl]acetic acid

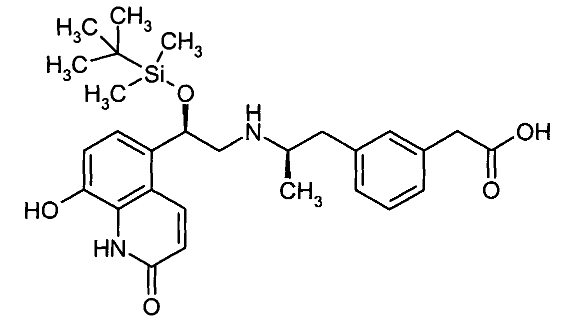

Lithium hydroxide solution (4.23ml, 1M, 4.23mmol) was added to a solution of the compound from preparation 21 (1.11g, 2.16mmol) in tetrahydrofuran (10ml) and the reaction stirred at room temperature for 18 hours. Tlc analysis showed starting material remaining, so additional lithium hydroxide (2.1ml, 1M, 2.1mmol) was added and the reaction stirred for a further 18 hours at room temperature and at 45°C for a further 5 hours. The mixture was acidifed by the addition of 1M hydrochloric acid (6.33ml), then concentrated under reduced pressure to remove tetrahydrofuran. The remaining aqueous solution was decanted off, and the residue azeotroped with methanol to afford the title compound as a brown foam, 1.1g.
¹H nmr (CD₃OD, 400MHz) δ: -0.18 (s, 3H), 0.10 (s, 3H), 0.85 (s, 9H), 1.23 (d, 3H), 2.78 (m, 1H), 3.00 (m, 1H), 3.25 (dd, 1H), 3.38 (m, 1H), 3.42-3.58 (m, 3H), 5.49 (m, 1H), 6.67 (d, 1H), 7.05 (m, 2H), 7.13 (s, 1H), 7.22 (m, 3H), 8.40 (d, 1H).
LRMS : m/z ES⁺ 511 [MH]⁺

### Preparation 25

### [3-(2-{[(2R)-2-{[tert-Butyl(dimethyl)silyl]oxy}-2-(8-hydroxy-2-oxo-1,2-dihydroquinolin-5-yl)ethyl]amino}-2-methylpropyl)phenyl]acetic acid

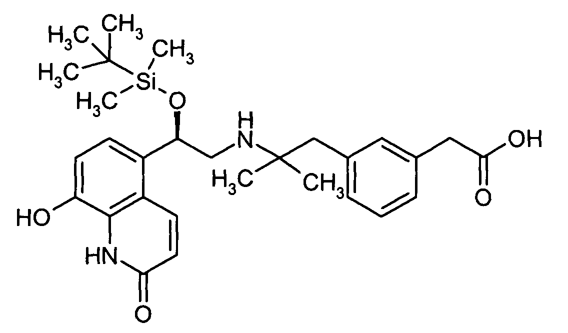

A mixture of the ester from preparation 22 (1.85g, 3.43mmol) and aqueous lithium hydroxide solution (10.3ml, 1M, 10.3mmol) in tetrahydrofuran (15ml) was stirred at room temperature for 18 hours. Tlc analysis showed starting material remaining, so additional lithium hydroxide (3.5ml, 1M, 3.5mmol) was added and the mixture stirred for a further 18 hours. The mixture was neutralised using 1M hydrochloric acid (13.8ml, 1M, 13.8mmol) and the tetrahydrofuran removed under reduced pressure. The resulting suspension was stirred for 30 minutes and the precipitate filtered off, washed with water (10ml), and ether (40ml) and dried *in vacuo* to afford the title compound as a beige solid, 1.78g.
¹H nmr (CD₃OD, 400MHz) δ: -0.17 (s, 3H), 0.08 (s, 3H), 0.83 (s, 9H), 1.26 (s, 3H), 1.28 (s, 3H), 1.86 (m, 1H), 2.89-2.96 (m, 2H), 3.54 (s, 2H), 3.71 (m, 1H), 5.42 (m, 1H), 6.70 (d, 1H), 7.01 (d, 1H), 7.06 (d, 1H), 7.12 (s, 1H), 7.17 (d, 1H), 7.23-7.30 (m, 2H), 8.38 (d, 1H).
LRMS : m/z ES⁺ 525 [MH]⁺

### Preparations 26 to 35

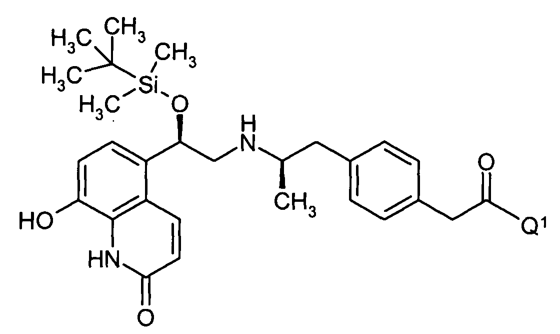

1-(3-Dimethylaminopropyl)-3-ethylcarbodiimide hydrochloride (53mg, 0.28mmol), was added to a solution of the acid from preparation 23 (140mg, 0.27mmol), 1-hydroxybenzotriazole hydrate (39mg, 0.29mmol) and triethylamine (0.11ml, 0.79mmol) in N,N-dimethylformamide (3ml). A solution of the appropriate amine (RNH₂) (0.28mmol) in N,N-dimethylformamide (2ml) was added and the reaction stirred at room temperature for 72 hours. The reaction mixture was concentrated under reduced pressure and the residue diluted with dichloromethane, and washed with sodium bicarbonate solution (10ml) and brine (2x10ml). The organic phase was dried (Na₂SO₄) and evaporated under reduced pressure. The crude product was purified by column chromatography on silica gel using an elution gradient of dichloromethane:methanol:0.88 ammonia (99.8:0:0.2 to 94.8:4:0.2). The resulting oil was azeotroped with ether to afford the title compound as a foam.

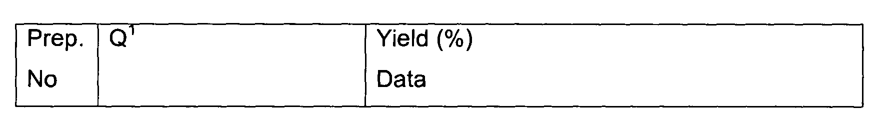

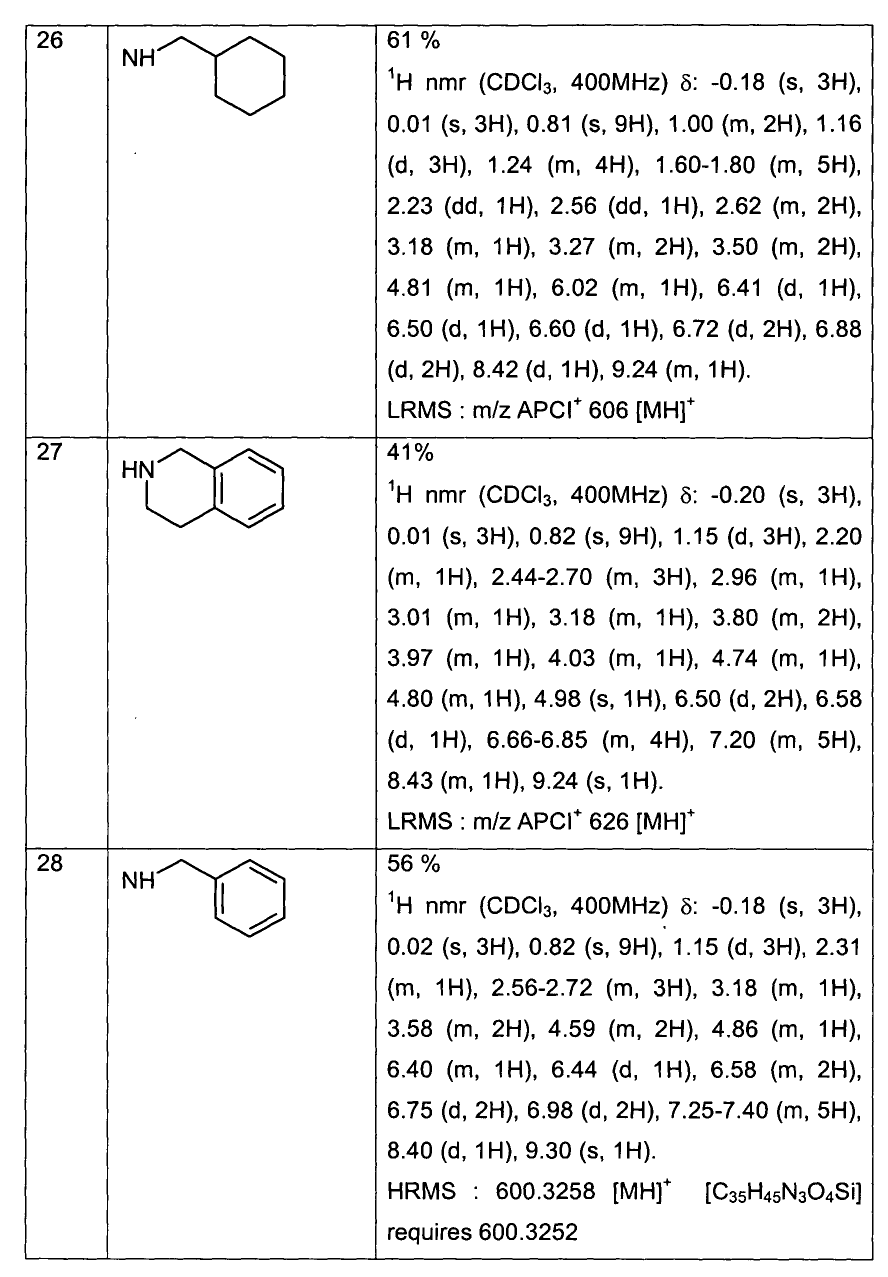

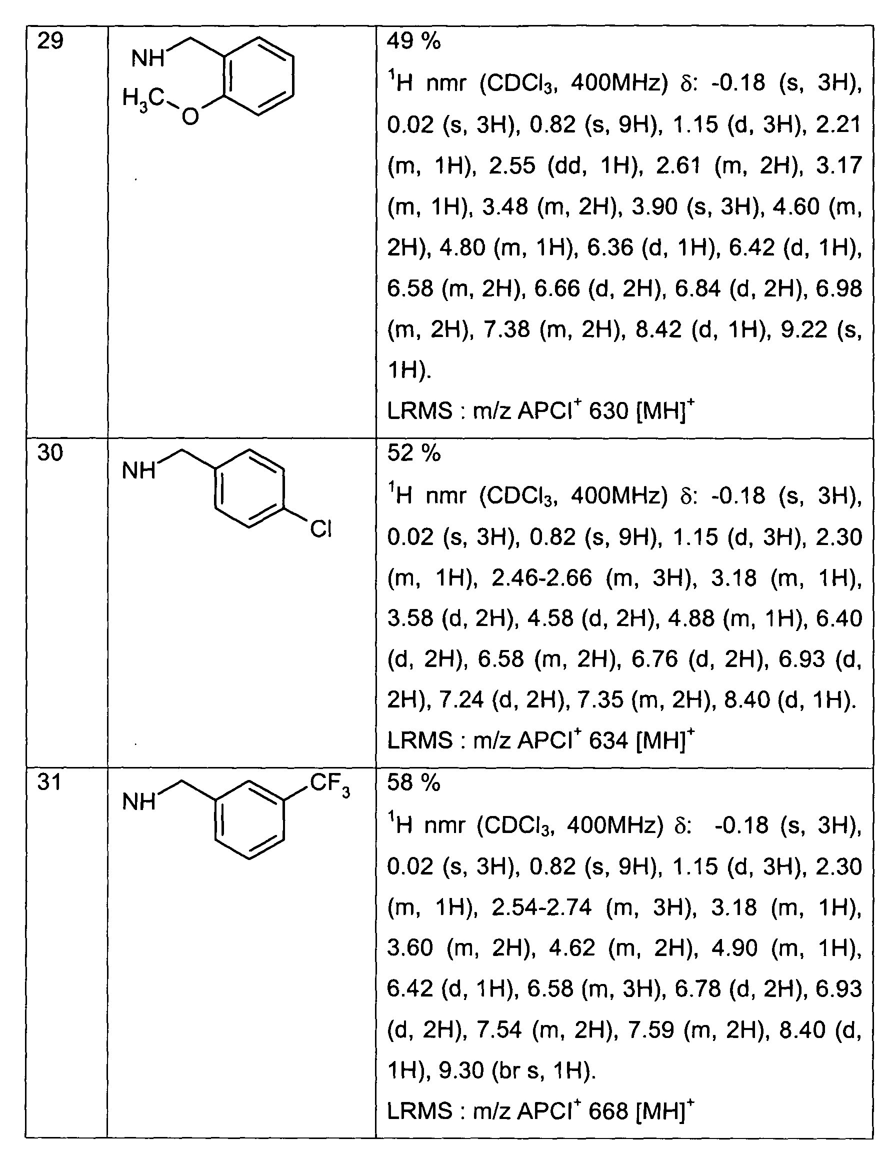

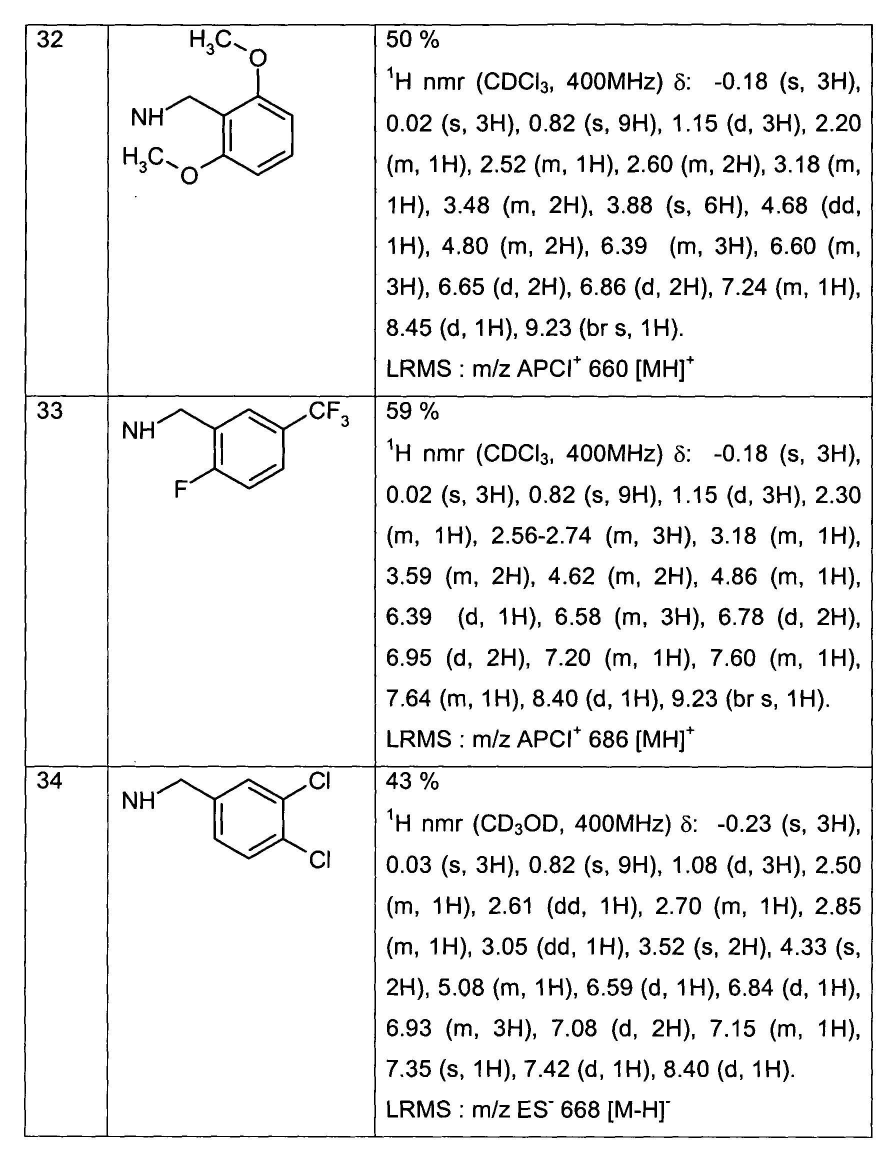

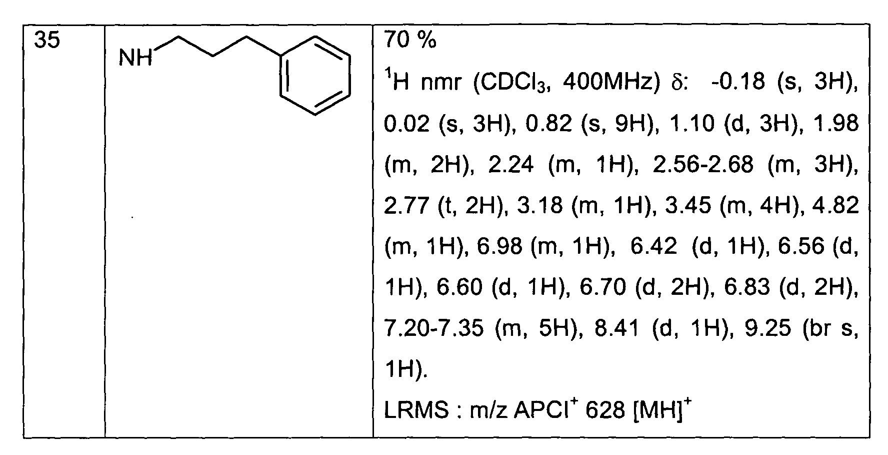

### Preparations 36 to 45

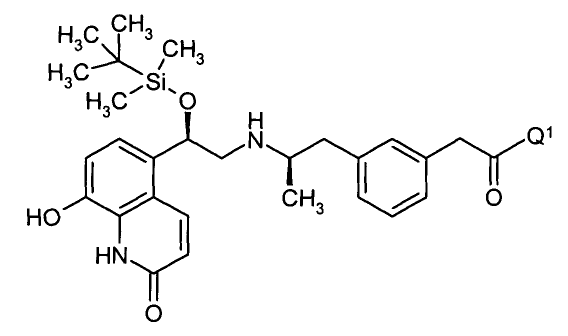

1-(3-Dimethylaminopropyl)-3-ethylcarbodiimide hydrochloride (1.2eq), was added to a solution of the acid from preparation 24 (1eq), 1-hydroxybenzotriazole hydrate (1.2eq) the appropriate amine (RNH₂) (1.2eq) and triethylamine (3eq) in N,N-dimethylformamide (7-15ml/mmol) and the reaction stirred at room temperature for 72 hours. The mixture was evaporated under reduced pressure. The crude product was purified by column chromatography on silica gel using an elution gradient of dichloromethane:methanol:0.88 ammonia (98:2:0.2 to 95:5:0.5) to afford the title compound.

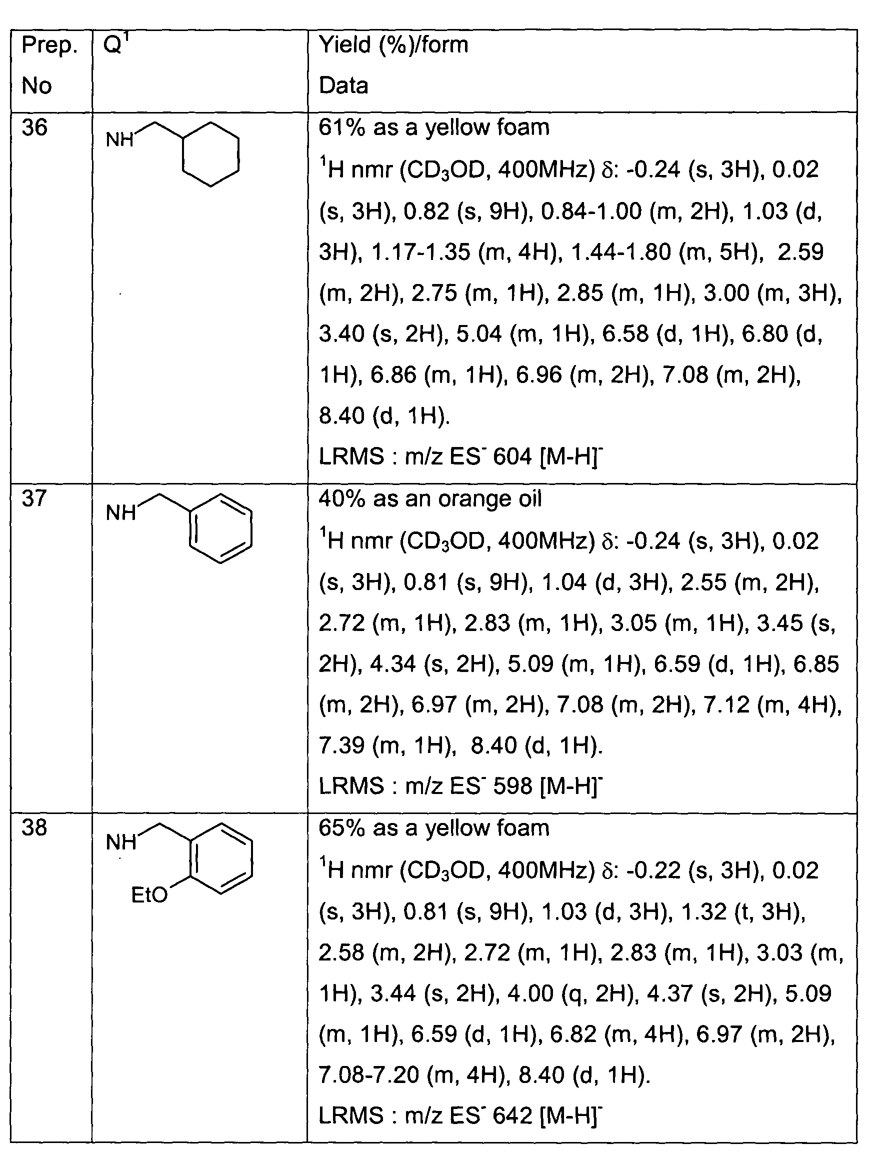

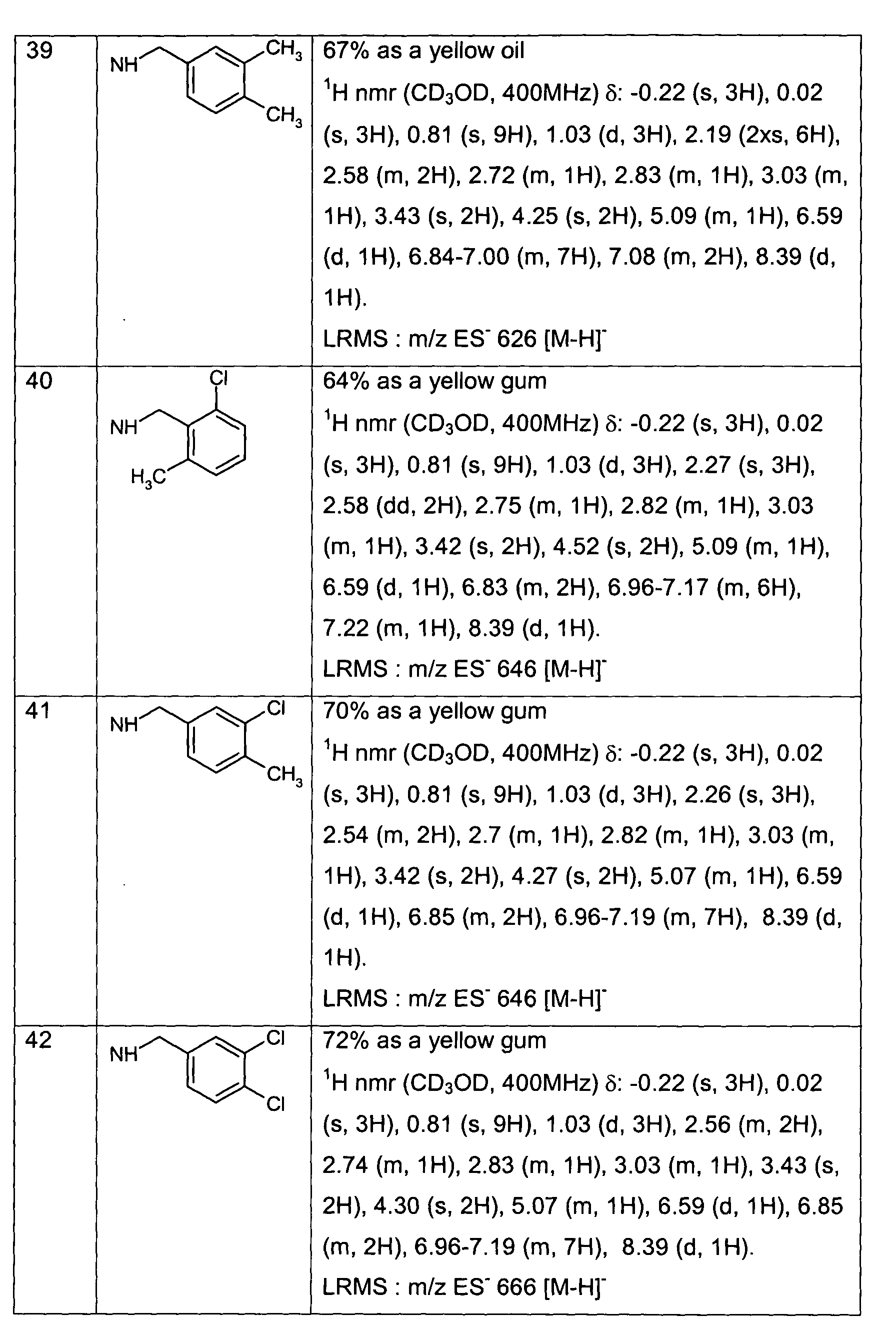

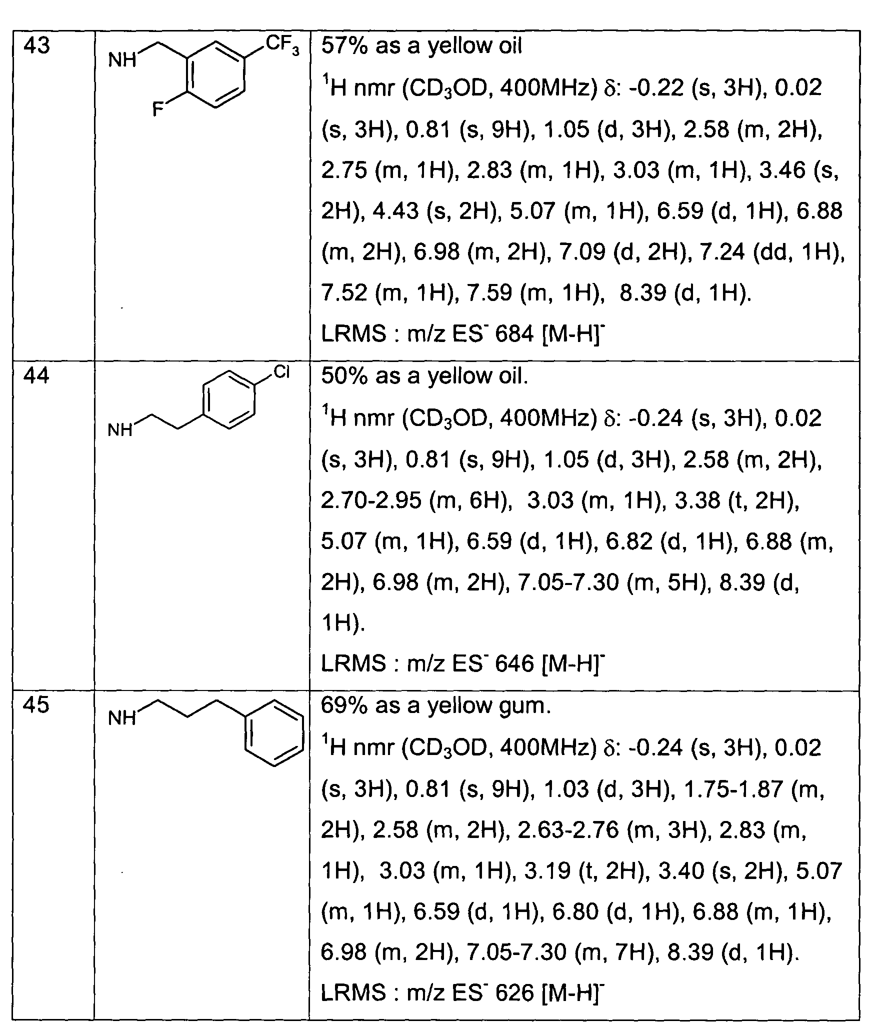

### Preparations 46 to 56

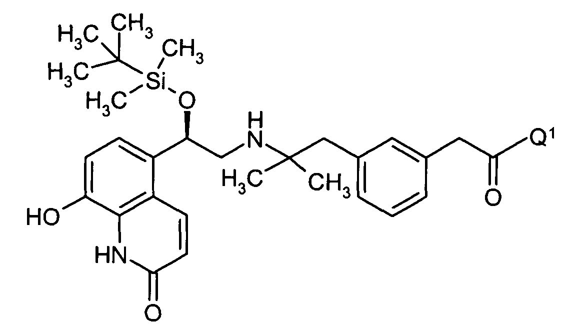

1-(3-Dimethylaminopropyl)-3-ethylcarbodiimide hydrochloride (1.2-1.4eq), was added to a solution of the acid from preparation 25 (1eq), the appropriate amine (RNH₂) (1.2-2.1eq), 1-hydroxybenzotriazole hydrate (1.1-1.4eq) and triethylamine (2.0-2.1eq) in N,N-dimethylformamide (11ml/mmol), and the reaction stirred at room temperature for 18 hours. The reaction mixture was concentrated under reduced pressure and the residue diluted with sodium bicarbonate solution (10ml) and this mixture extracted with dichloromethane (4x10ml). The combined organic extracts were washed with brine (5ml), dried (Na₂SO₄) and evaporated under reduced pressure. The crude product was purified by column chromatography on silica gel using dichloromethane:methanol (95:5) as eluant to afford the title compound.

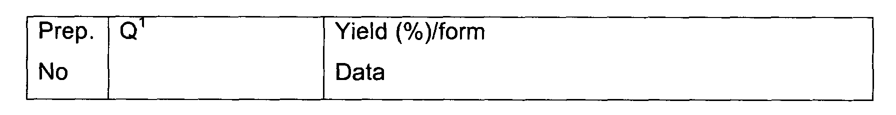

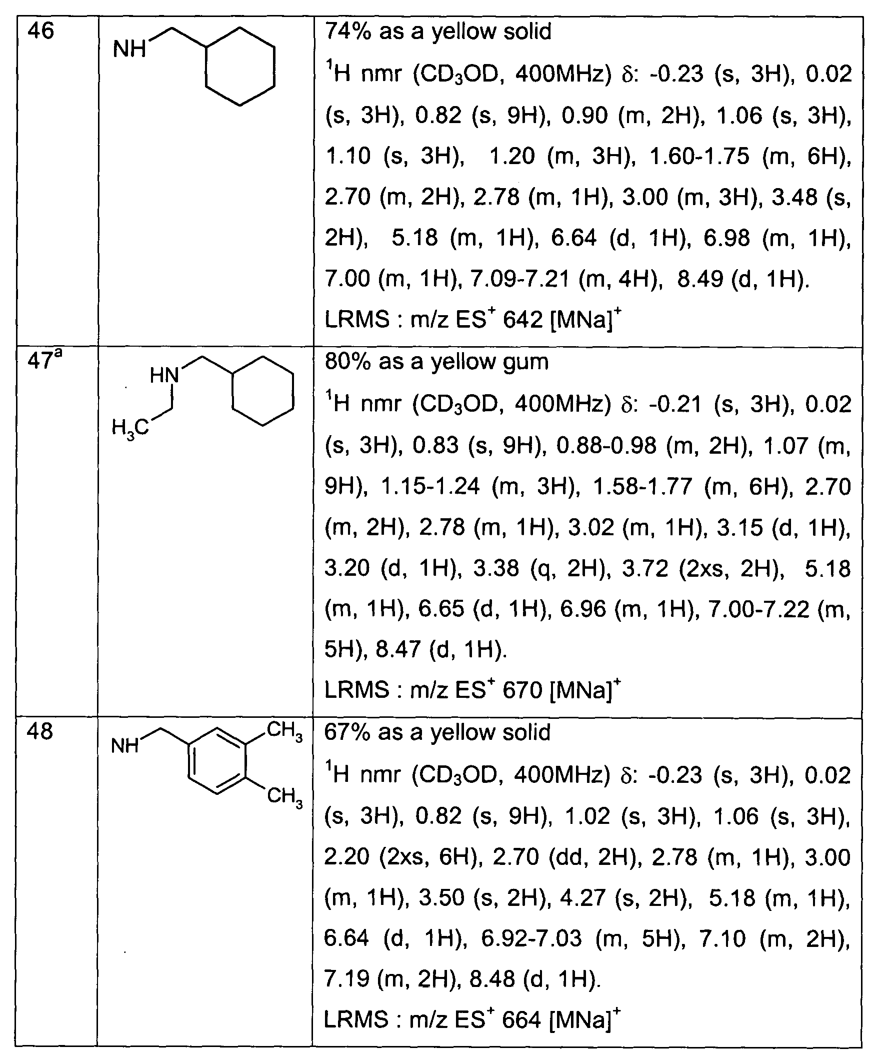

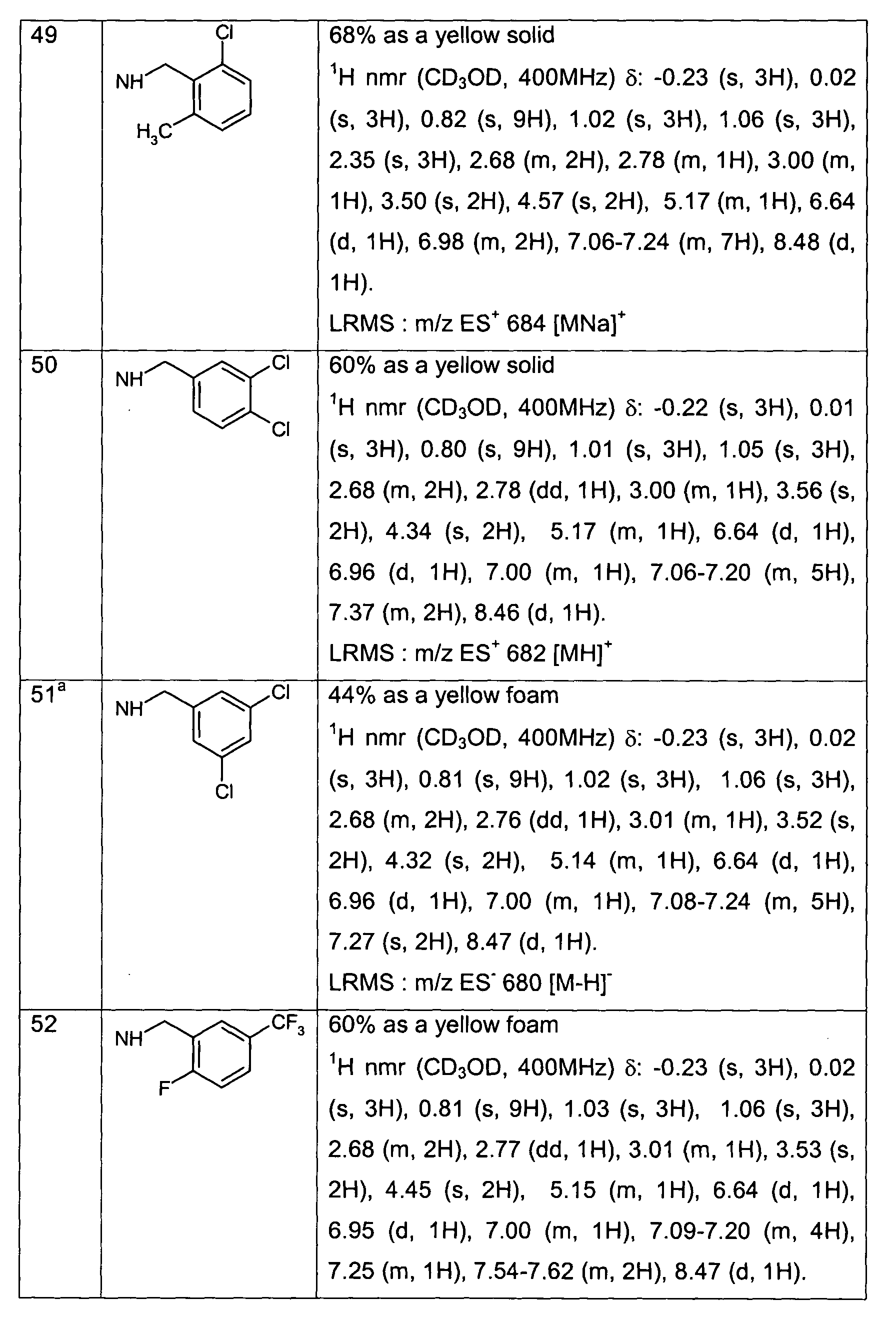

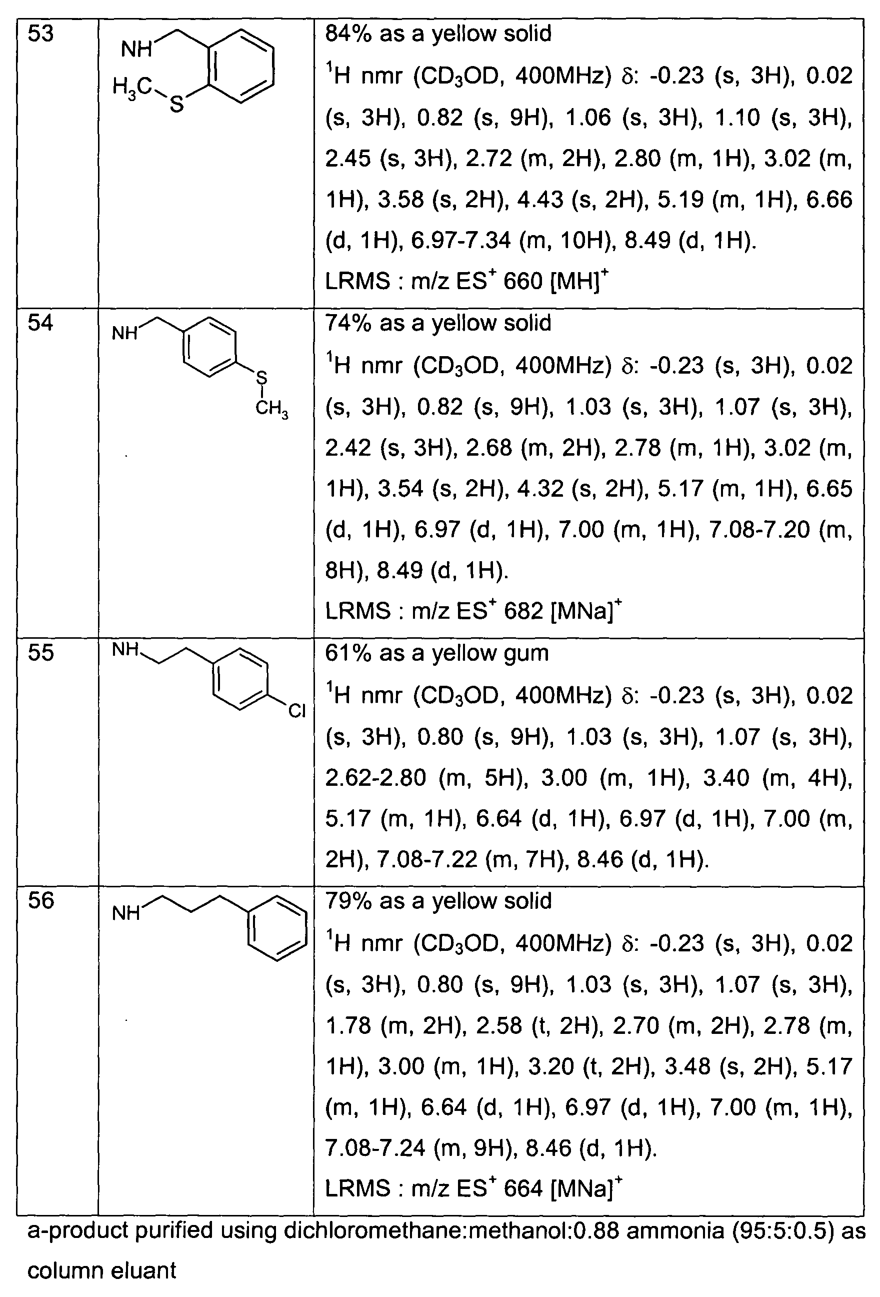

### Examples 1 to 10

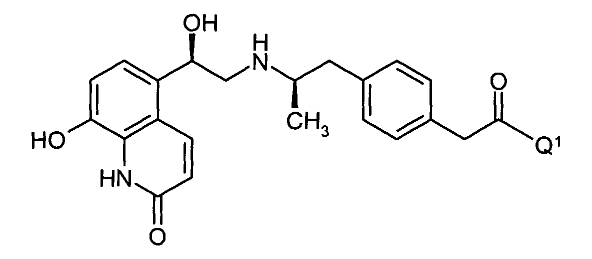

A mixture of the silyl protected compound (from preparations 26 to 35) (1eq) and ammonium formate (10eq) in methanol (20-30ml/mmol) and water (11-15ml/mmol) was stirred at 45°C for 18 hours. The reaction mixture was evaporated under reduced pressure and the residue purified by column chromatography on silica gel using an elution gradient of dichloromethane:methanol:0.88 ammonia (94.7:5:0.3 to 89.7:10:0.3). The residue was azeotroped with methanol to afford the title compound as an oil/foam.

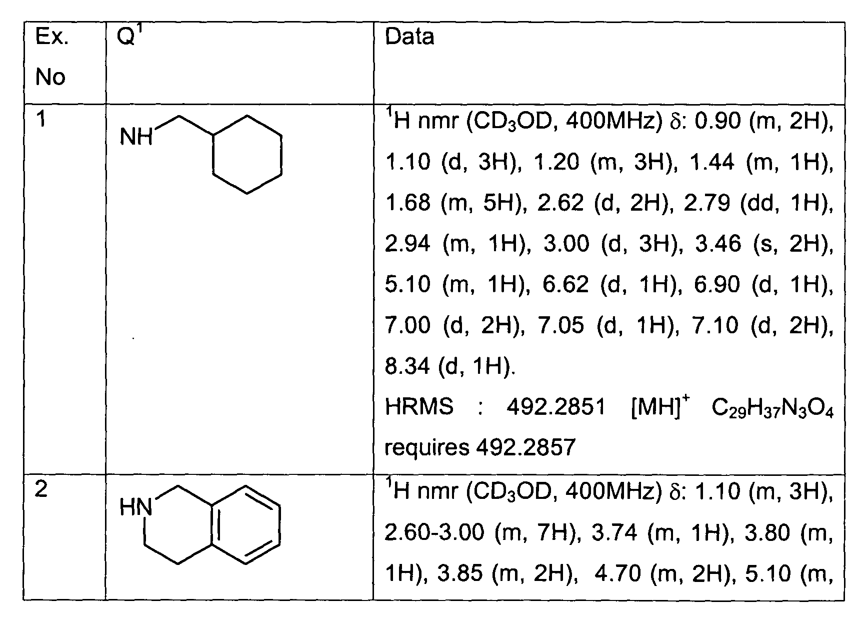

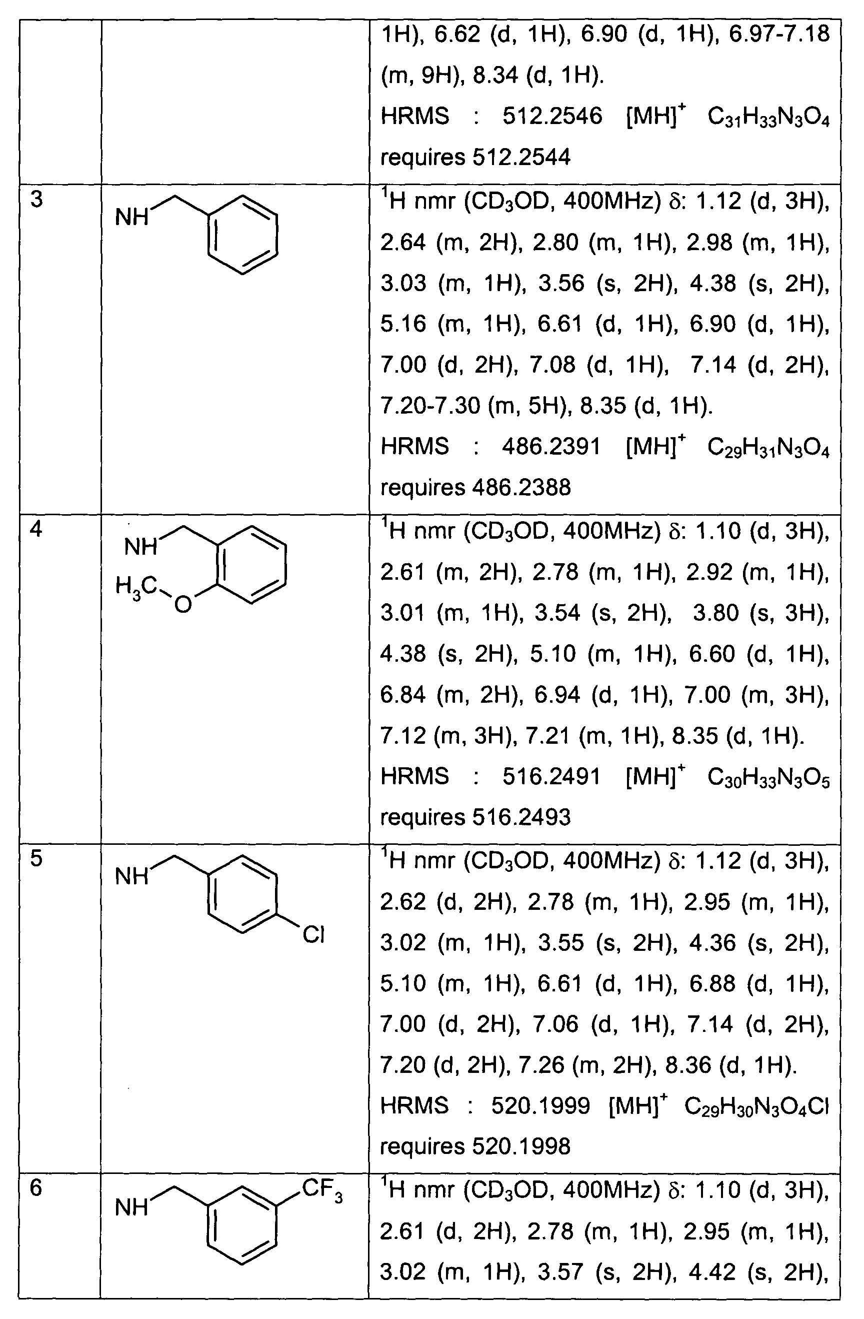

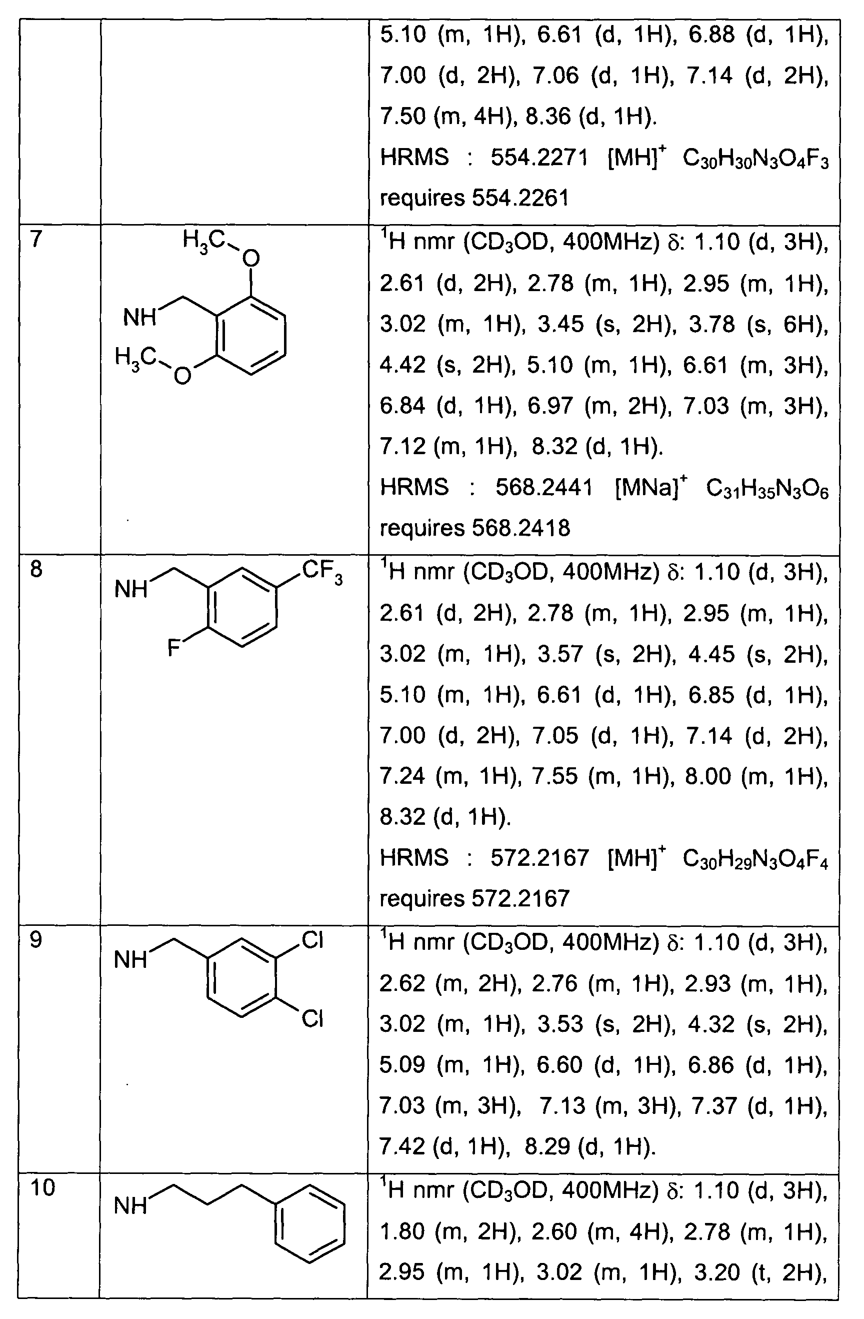

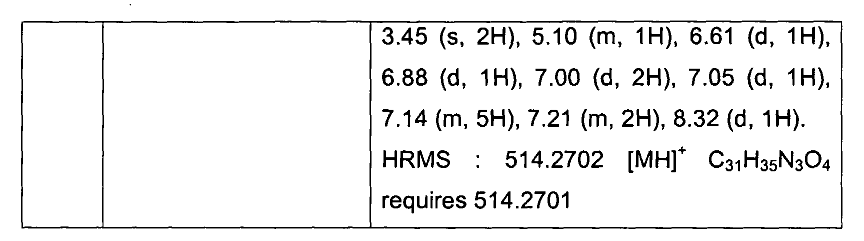

### Examples 11 to 20

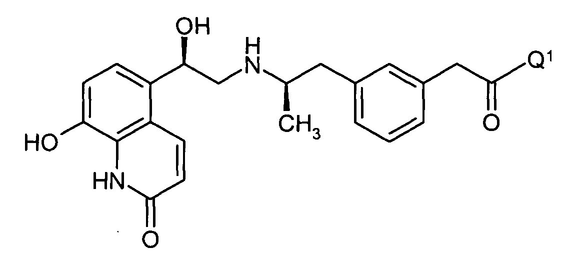

A mixture of the silyl protected compound (from preparations 36 to 45) (1eq) and ammonium formate (10eq) in methanol (40ml/mmol) and water (24ml/mmol) was stirred at 40°C for 18-42 hours. The reaction mixture was evaporated under reduced pressure and the residue purified by column chromatography on silica gel using an elution gradient of dichloromethane:methanol:0.88 ammonia (95:5:0.5 to 92:8:0.8) to afford the title compound.

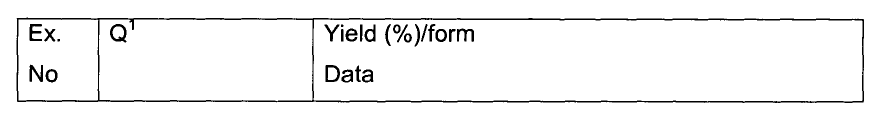

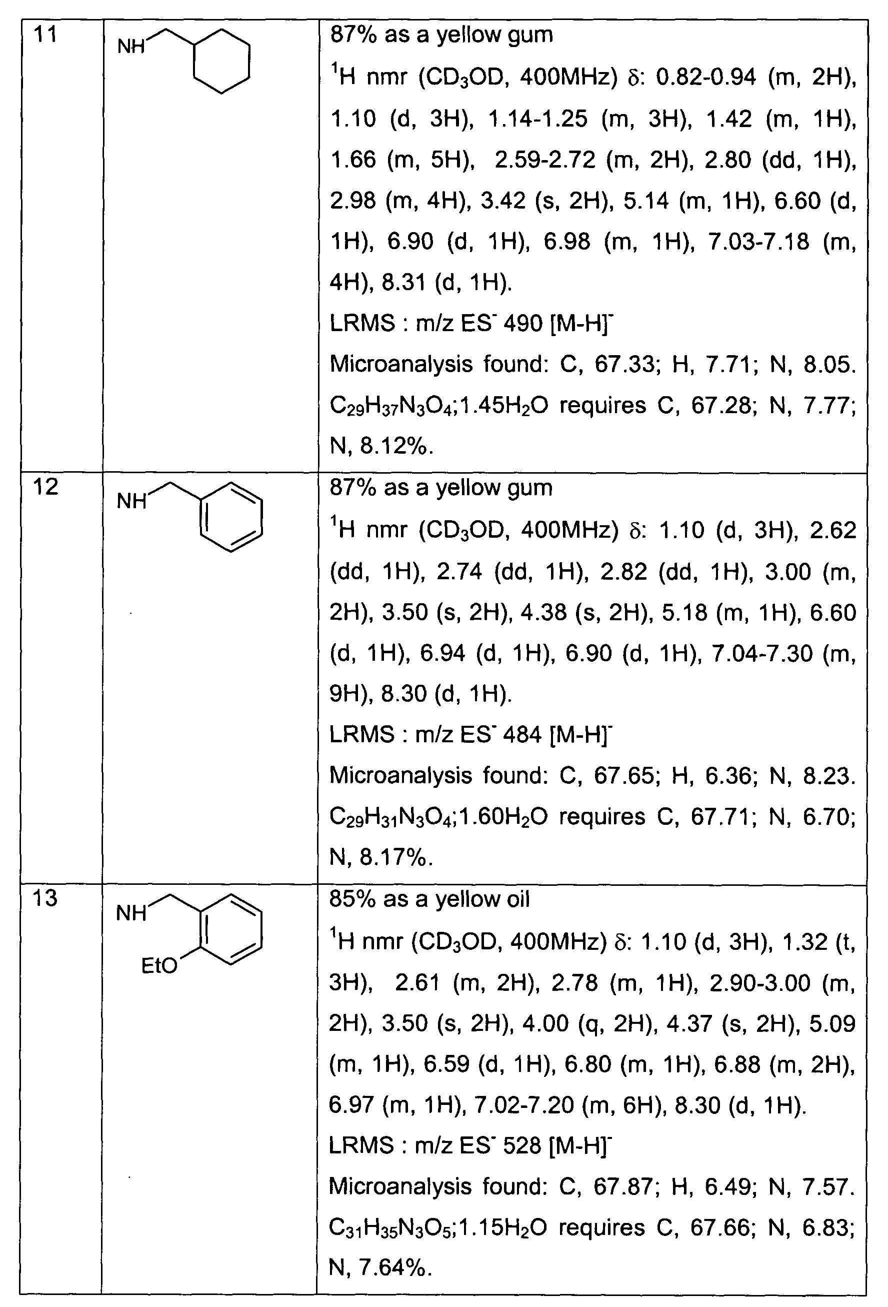

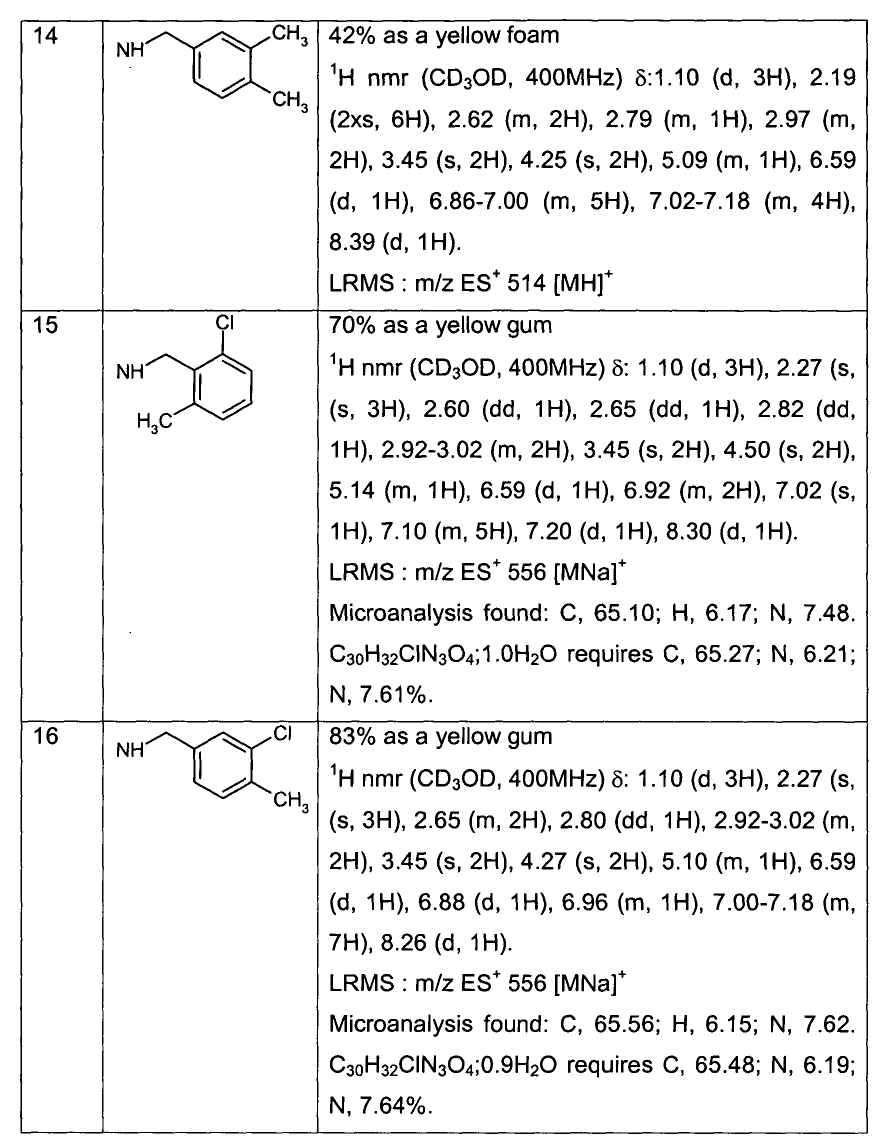

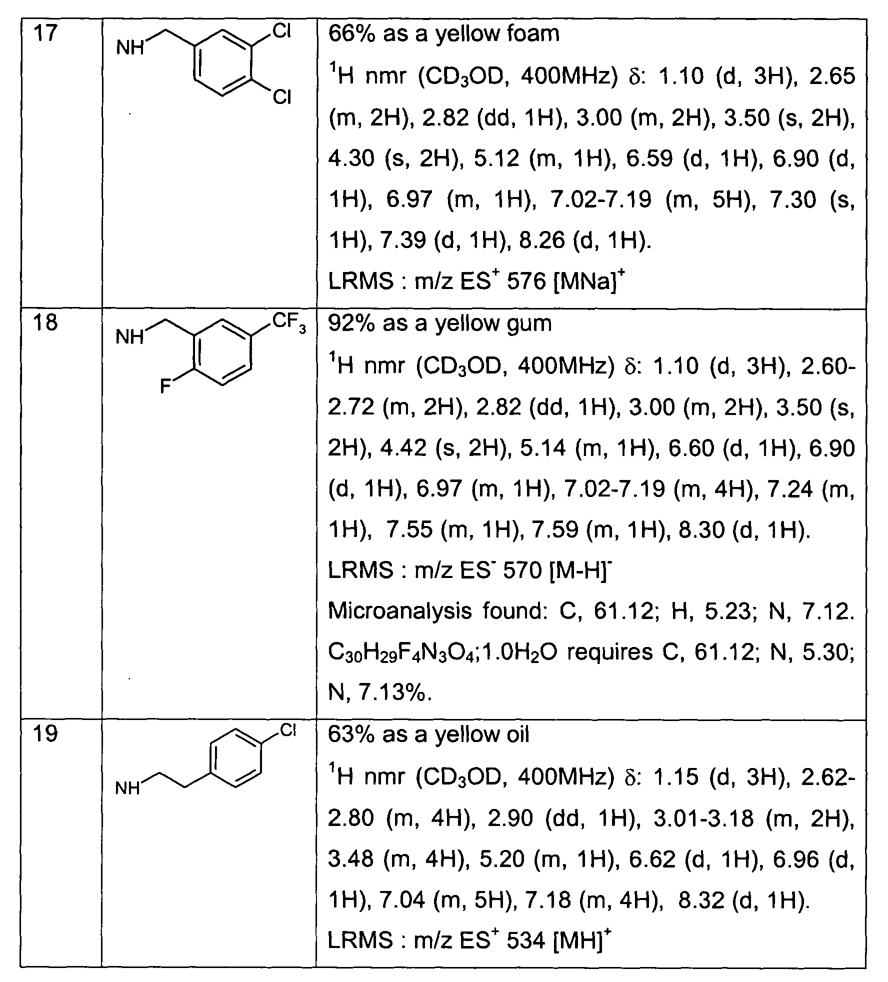

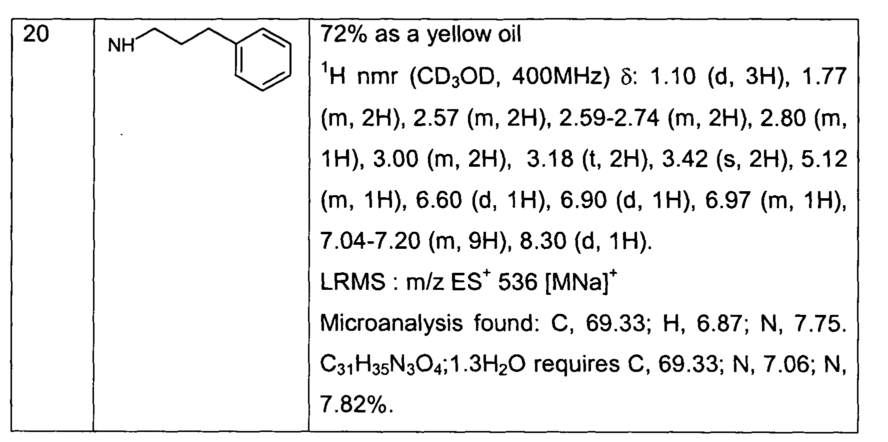

### Examples 21 to 31

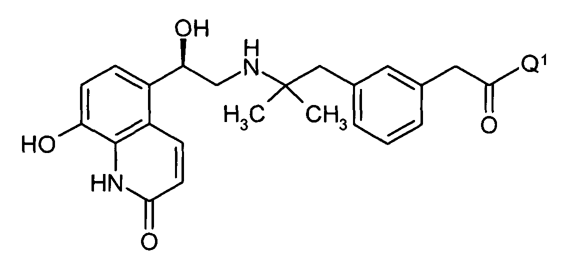

A mixture of the silyl protected compound (from preparations 46 to 56) (1eq) and ammonium formate (10-18eq) in methanol (50ml/mmol) and water (33ml/mmol) was stirred at 40°C for 18 hours. The reaction mixture was concentrated under reduced pressure and the residue azeotroped with dichloromethane:methanol (9:1 by volume) or toluene. The crude product was triturated with ether, the resulting solid filtered off, washed with water and ether and dried *in vacuo* to afford the title compound.

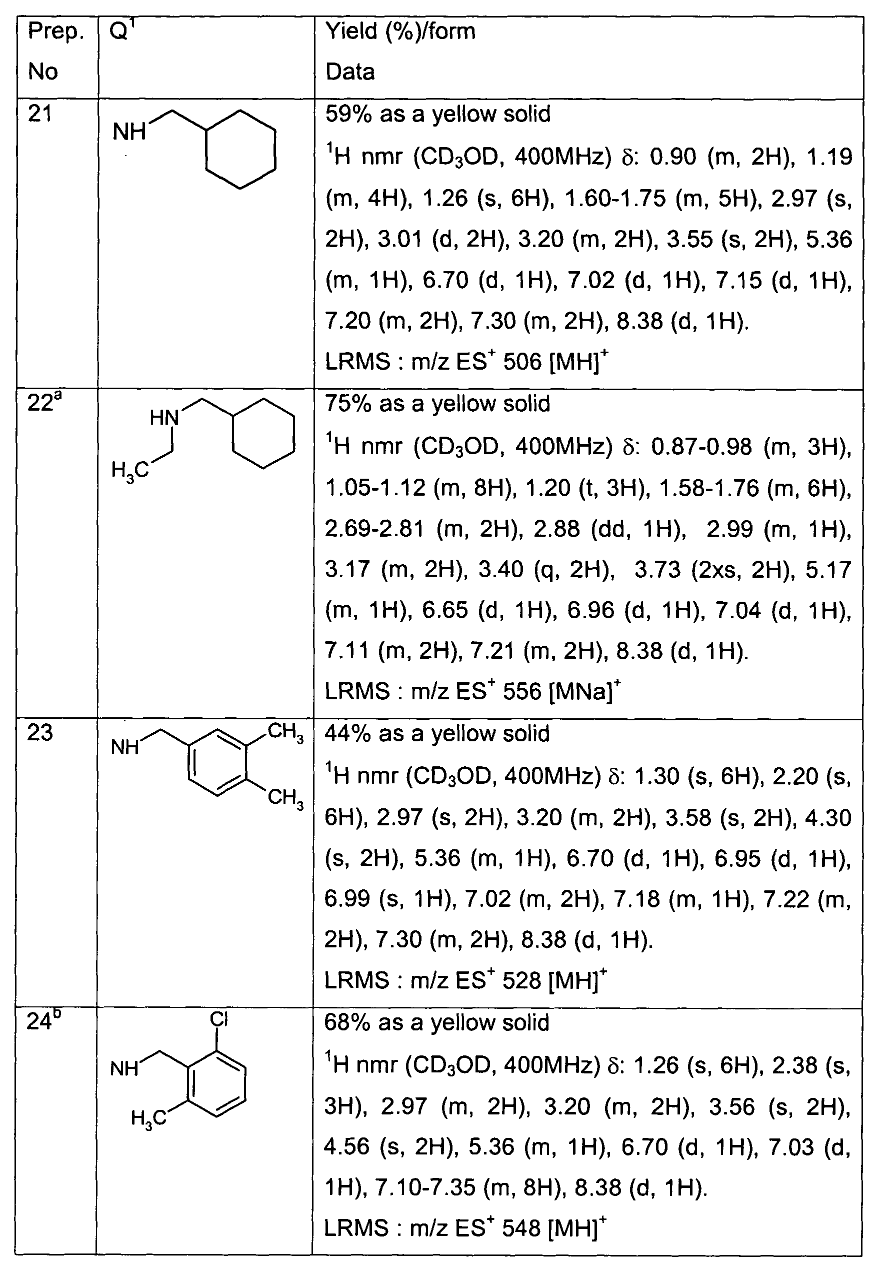

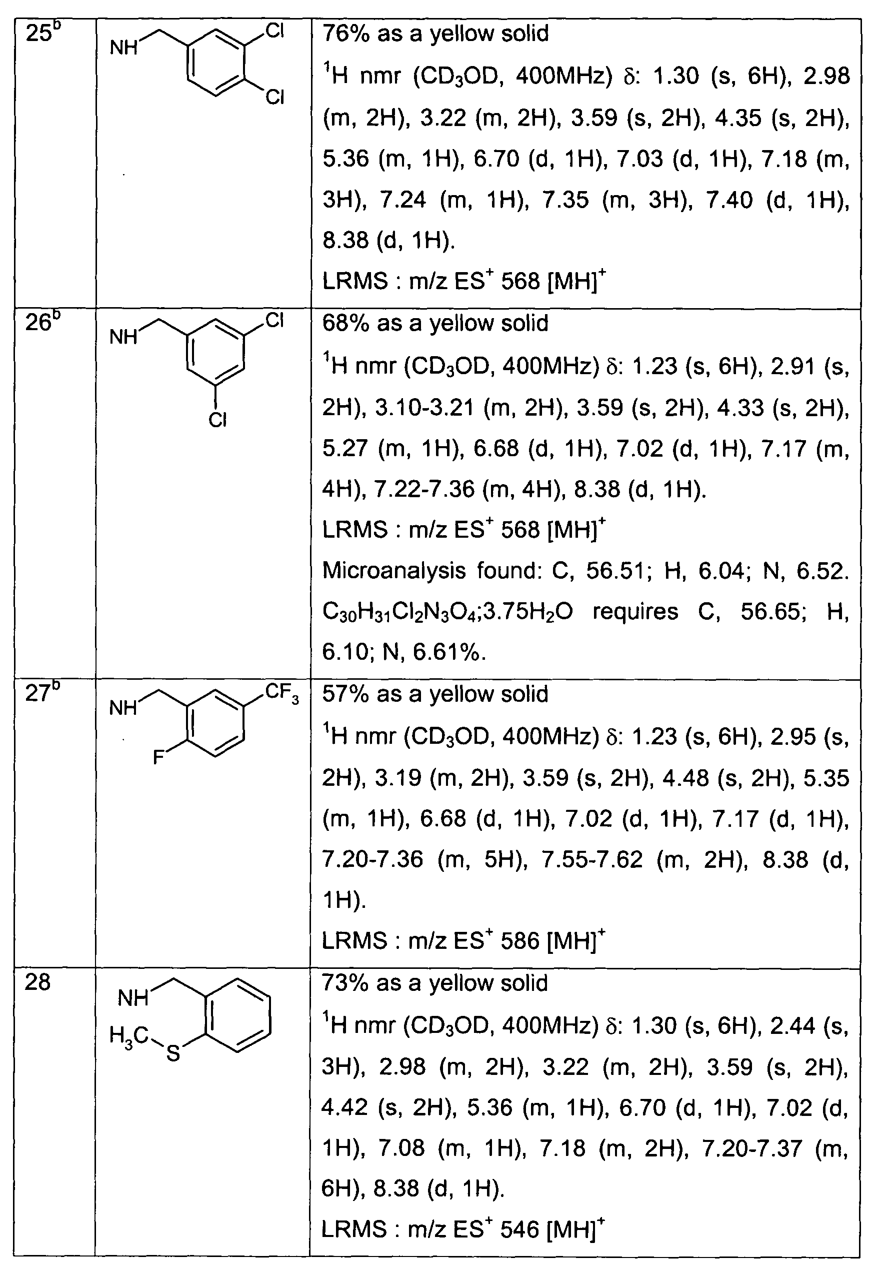

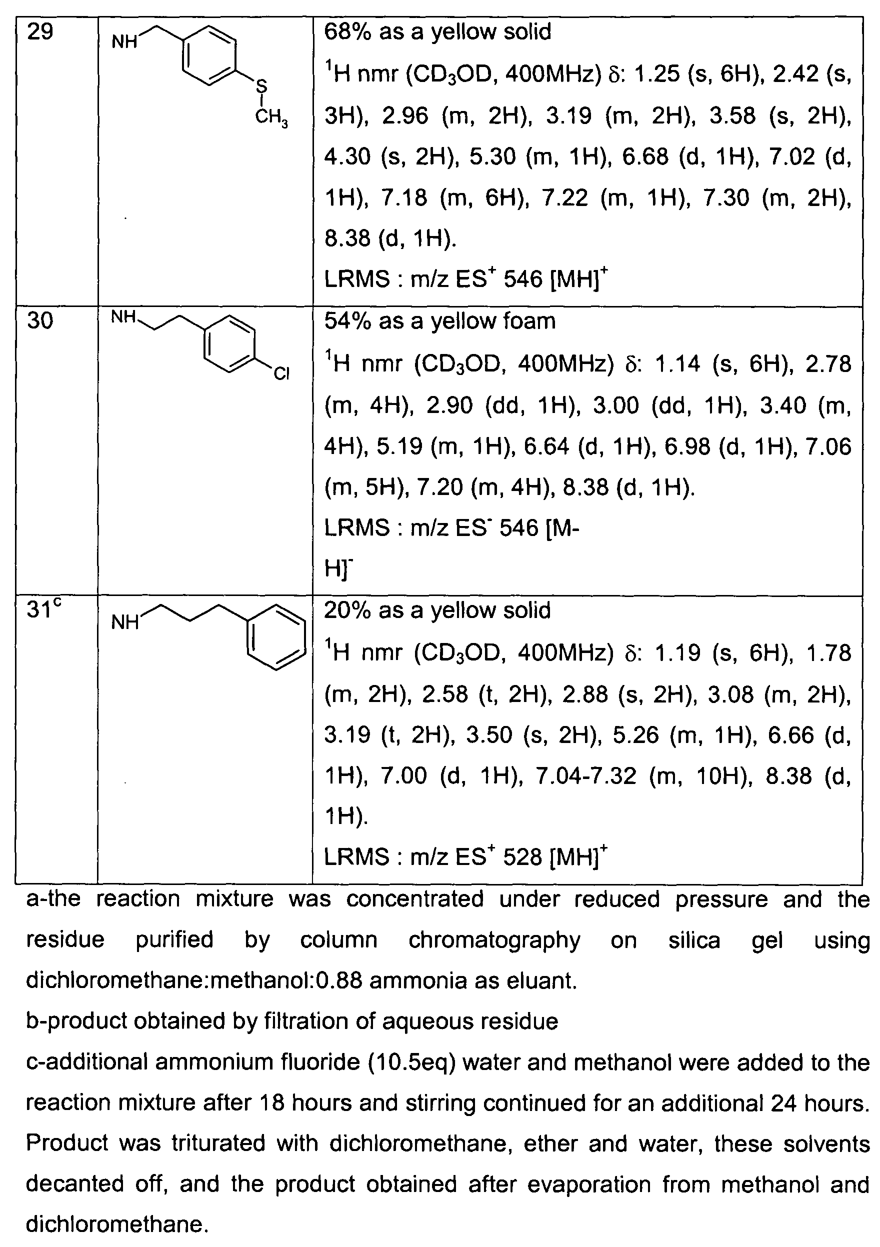

The ability of the compounds of the formula (1) to act as potent β2 agonists therefore mediating smooth muscle relaxation may be determined by the measure of the effect of beta-2 adrenergic receptor stimulation on electrical field stimulated-contraction of guinea pig trachea strips.

### Guinea-pig trachea

Male, Dunkin-Hartley guinea pigs (475-525g) are killed by CO₂ asphyxiation and exsanguination from the femoral artery and the trachea is isolated. Four preparations are obtained from each animal, starting the dissection immediately below the larynx and taking 2.5 cm length of trachea. The piece of trachea is opened by cutting the cartilage opposite the trachealis muscle, then transverse sections, 3-4 cartilage rings wide, are cut. The resulting strip preparations are suspended in 5 ml organ baths using cotton threads tied through the upper and lower cartilage bands. The strips are equilibrated, un-tensioned, for 20 minutes in a modified Krebs Ringer buffer (Sigma K0507) containing 3 µM Indomethacin (Sigma 17378), 10 µM Guanethidine (Sigma G8520) and 10 µM Atenolol (Sigma A7655), heated at 37°C and gassed with 95% O₂/5% CO₂, before applying an initial tension of 1 g. The preparations are allowed to equilibrate for a further 30-45 minutes, during which time they are re-tensioned (to 1 g) twice at 15-minute intervals. Changes in tension are recorded and monitored via standard isometric transducers coupled to a data-collection system (custom-designed at Pfizer). Following the tensioning equilibration, the tissues are subjected to electrical field stimulation (EFS) using the following parameters : 10 s trains every 2 minutes, 0.1 ms pulse width, 10 Hz and just-maximal voltage (25 Volts) continuously throughout the length of the experiment. EFS of post-ganglionic cholinergic nerves in the trachea results in monophasic contractions of the smooth muscle and twitch height is recorded. The organ baths are constantly perfused with the above-described Krebs Ringer buffer by means of a peristaltic pump system (pump flow rate 7.5 ml / minute) throughout the experiment, with the exception of when a beta-2 agonist according to the present invention is added, the pump is then stopped for the time of the cumulative dosing to the bath and started again after maximal response is reached for the wash-out period.

### Experimental protocol for assessment of potency and efficacy

Following equilibration to EFS, the peristaltic pump is stopped and the preparations 'primed' with a single dose of 300 nM isoprenaline (Sigma 15627) to establish a maximal response in terms of inhibition of the contractile EFS response. The isoprenaline is then washed out over a period of 40 minutes. Following the priming and wash-out recovery, a standard curve to isoprenaline is carried out on all tissues (Isoprenaline Curve 1) by means of cumulative, bolus addition to the bath using half log increments in concentration. The concentration range used is 1^{e-9} to 1^{e}/3^{e-6} M. At the end of the isoprenaline curve the preparations are washed again for 40 minutes before commencing a second curve, either to isoprenaline (as internal control) or a beta-2 agonist according to the present invention. Beta-2 agonist responses are expressed as percentage inhibition of the EFS response. Data for beta-2 agonist are normalised by expressing inhibition as a percentage of the maximal inhibition induced by isoprenaline in Curve 1. The EC₅₀ value for beta-2 agonist according to the present invention refers to the concentration of compound required to produce half maximal effect. Data for beta-2 agonists according to the present invention are then expressed as relative potency to isoprenaline defined by the ratio (EC₅₀ beta-2 agonist)/(EC50 Isoprenaline).

### Confirmation of beta-2 mediated functional activity

Beta-2 agonist activity of test compounds is confirmed using the protocol above, however, prior to constructing the curve to beta-2 agonist according to the present invention, the preparations are pre-incubated (for a minimum of 45 minutes) with 300 nM ICI 118551 (a selective β2 antagonist) which results in the case of a beta-2 mediated effect in a rightward-shift of the test compound dose response curve.

According to another alternative, the agonist potency for the β2 receptor of the compounds of the formula (1) may also be determined by the measure of the concentration of compound according to the present invention required to produce half maximal effect (EC₅₀) for the β2 receptor.

### Compound Preparation

10 mM/100% DMSO (dimethylsulfoxide) stock of compound is diluted to required top dose in 4 % DMSO. This top dose is used to construct a 10-point semi-log dilution curve, all in 4 % DMSO. Isoprenaline (Sigma, I-5627) was used as a standard in every experiment and for control wells on each plate. Data was expressed as % Isoprenaline response.

### Cell Culture

CHO (Chinese Hamster Ovary) cells recombinantly expressing the human β2 adrenergic receptor (from Kobilka et al., PNAS 84: 46-50, 1987 and Bouvier et al., Mol Pharmacol 33: 133-139 1988 CHOhβ2) were grown in Dulbeccos MEM/NUT MIX F12 (Gibco, 21331-020) supplemented with 10 % foetal bovine serum (Sigma, F4135, Lot 90K8404 Exp 09/04), 2 mM glutamine (Sigma, G7513), 500 µg/ml geneticin (Sigma, G7034) and 10 µg/ml puromycin (Sigma, P8833). Cells were seeded to give about 90 % confluency for testing.

### Assay Method

25 µl / well each dose of compound was transferred into a cAMP- Flashplate® (NEN, SMP004B), with 1% DMSO as basal controls and 100 nM Isoprenaline as max controls. This was diluted 1:2 by the addition of 25 µl / well PBS. Cells were trypsinised (0.25% Sigma, T4049), washed with PBS (Gibco, 14040-174) and resuspended in stimulation buffer (NEN, SMP004B) to give 1x10⁶ cells / ml CHOhB2. Compounds were incubated with 50 µl / well cells for 1 hour. Cells were then lysed by the addition of 100 µl / well detection buffer (NEN, SMP004B) containing 0.18 µCi / ml ¹²⁵I-cAMP (NEN, NEX-130) and plates were incubated at room temperature for a further 2 hours. The amount of ¹²⁵I-cAMP bound to the Flashplate® was quantified using a Topcount NXT (Packard), normal counting efficiency for 1 minute. Dose-response data was expressed as % Isoprenaline activity and fitted using a four parameter sigmoid fit.

It has thus been found that the compounds of formula (1) according to the present invention that are illustrated in examples 1 to 31 above show a β2 cAMP EC₅₀ below 1nM.

## Claims

1. A compound of general formula(1 , 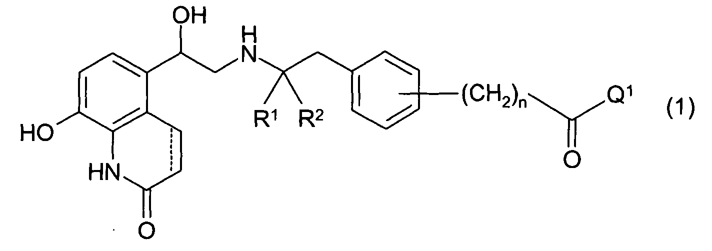 wherein the dashed line represents an optional bond, the (CH₂)ₙ-C(=O)Q¹ group is in the meta or para position,
- R¹ and R² are independently selected from H and C₁-C₄ alkyl,
- n is 0, 1 or 2,
- Q¹ is a group selected from, 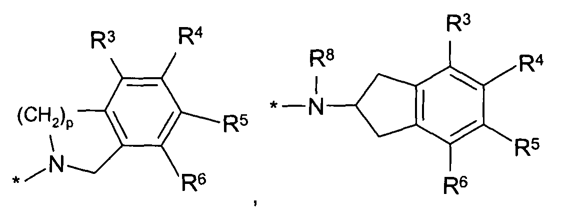 and a group *-NR⁸-Q²-A, wherein p is 1 or 2, Q² is a C₁-C₄ alkylene, R⁸ is H or C₁-C₄ alkyl and A is pyridyl, C₃-C₇ cycloalkyl, tetrahydropyranyl, piperidinyl, tetrahydrothiopyranyl or a group 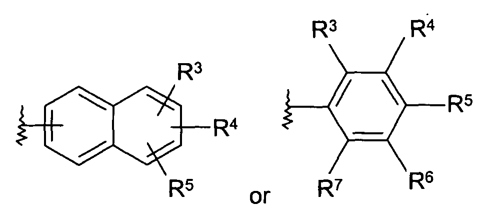
- R³, R⁴, R⁵, R⁶ and R⁷ are the same or different and are selected from H, C₁-C₄ alkyl, OR⁹, SR⁹, halo, CF₃, OCF₃, SO₂NR⁹R¹⁰, CONR⁹R¹⁰, NR⁹R¹⁰, NHCOR¹¹ and phenyl;
- R¹¹ is C₁-C₄ alkyl and R⁹ and R¹⁰ are the same or different and are selected from H or C₁-C₄ alkyl and the * represent the attachment point to the carbonyl group;
or, if appropriate, their pharmaceutically acceptable salts and/or isomers, tautomers, solvates or isotopic variations thereof.

2. A compound according to claim 1 wherein Q¹ is a group *-NH-Q²-A, wherein A is cyclohexyl.

3. A compound according to claim 1 wherein Q¹ is a group *-NH-Q²-A, wherein A is a group 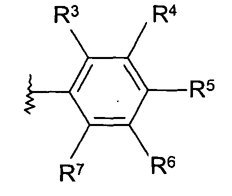 wherein R³, R⁴, R⁵, R⁶ and R⁷ are the same or different and are selected from H, C₁-C₄ alkyl, OR⁹, SR⁹, halo, CF₃, OCF₃, SO₂NR⁹R¹⁰, CONR⁹R¹⁰, NR⁹R¹⁰, NHCOR¹¹ and phenyl provided at least 2 of R³ to R⁷ are equal to H;
wherein R¹¹ is C₁-C₄ alkyl and R⁹ and R¹⁰ are the same or different and are selected from H or C₁-C₄ alkyl.

4. A compound according to claim 3 wherein Q¹ is a group *-NH-Q²-A, wherein A is a group 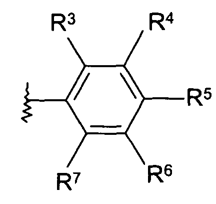 wherein R³, R⁴, R⁵, R⁶ and R⁷ are the same or different and are selected from H, CH₃, OCH₃, OCH₂-CH₃, SCH₃, halo, CF₃, provided at least 2 of R³ to R⁷ are equal to H.

5. A compound according to any one of claims 1 to 4 wherein Q² is -CH₂-,-(CH₂)₂-, -(CH₂)₃-, or (CH(CH₃)₂)-.

6. A compound according to claim 5 wherein Q² is -CH₂-.

7. A compound according to claim 1 wherein Q¹ is 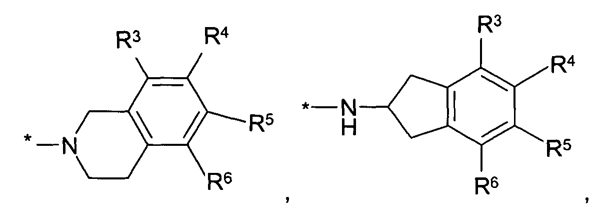 wherein R₃, R₄, R₅ and R₆ are H.

8. A compound according to any one of claims 1 to 7 wherein R¹ is H or C₁-C₄ alkyl and R² is C₁-C₄ alkyl.

9. A compound according to claim 8 wherein R¹ is H or CH₃ and R² is CH₃.

10. A compound according to any one of claim 1 to 9 wherein n is 0, 1, or 2.

11. A compound according to claim 10 wherein n is 1.

12. The (R,R)-stereoisomer of a compound according to any one of claims 1 to 11.

13. A compound according to any one of claim 1 to 12 wherein the (CH₂)ₙ-C(=O)Q¹ group is in the meta position.

14. A compound according to any one of claims 1 to 13 wherein the dashed line represents a bond.

15. A compound according to claim 1 selected from the group consisting of
*N*-(cyclohexylmethyl)-2-[4-((2*R*)-2-{[(2*R*)-2-hydroxy-2-(8-hydroxy-2-oxo-1,2-dihydroquinolin-5-yl)ethyl]amino}propyl)phenyl]acetamide;
5-{(1*R*)-2-[((1*R*)-2-{4-[2-(3,4-dihydroisoquinolin-2(1*H*)-yl)-2-oxoethyl]phenyl}-1-methylethyl)amino]-1-hydroxyethyl}-8-hydroxyquinolin-2(1*H*)-one;
*N*-benzyl-2-[4-((2*R*)-2-{[(2*R*)-2-hydroxy-2-(8-hydroxy-2-oxo-1,2-dihydroquinolin-5-yl)ethyl]amino}propyl)phenyl]acetamide;
2-[4-((2*R*)-2-{[(2*R*)-2-hydroxy-2-(8-hydroxy-2-oxo-1,2-dihydroquinolin-5-yl)ethyl]amino}propyl)phenyl]-*N*-(2-methoxybenzyl)acetamide;
*N*-(4-chlorobenzyl)-2-[4-((2*R*)-2-{[(2*R*)-2-hydroxy-2-(8-hydroxy-2-oxo-1,2-dihydroquinolin-5-yl)ethyl]amino}propyl)phenyl]acetamide;
2-[4-((2*R*)-2-{[(2*R*)-2-hydroxy-2-(8-hydroxy-2-oxo-1,2-dihydroquinolin-5-yl)ethyl]amino}propyl)phenyl]-*N*-[3-(trifluoromethyl)benzyl]acetamide;
*N*-(2,6-dimethoxybenzyl)-2-[4-((2*R*)-2-{[(2*R*)-2-hydroxy-2-(8-hydroxy-2-oxo-1,2-dihydroquinolin-5-yl)ethyl]amino}propyl)phenyl]acetamide;
*N*-[2-fluoro-5-(trifluoromethyl)benzyl]-2-[4-((2*R*)-2-{[(2*R*)-2-hydroxy-2-(8-hydroxy-2-oxo-1,2-dihydroquinolin-5-yl)ethyl]amino}propyl)phenyl]acetamide;
*N*-(3,4-dichlorobenzyl)-2-[4-((2*R*)-2-{[(2*R*)-2-hydroxy-2-(8-hydroxy-2-oxo-1,2-dihydroquinolin-5-yl)ethyl]amino}propyl)phenyl]acetamide;
2-[4-((2*R*)-2-{[(2*R*)-2-hydroxy-2-(8-hydroxy-2-oxo-1,2-dihydroquinolin-5-yl)ethyl]amino}propyl)phenyl]-*N*-(3-phenylpropyl)acetamide;
*N*-(cyclohexylmethyl)-2-[3-((2*R*)-2-{[(2*R*)-2-hydroxy-2-(8-hydroxy-2-oxo-1,2-dihydroquinolin-5-yl)ethyl]amino}propyl)phenyl]acetamide;
*N*-benzyl-2-[3-((2*R*)-2-{[(2*R*)-2-hydroxy-2-(8-hydroxy-2-oxo-1,2-dihydroquinolin-5-yl)ethyl]amino}propyl)phenyl]acetamide;
*N*-(2-ethoxybenzyl)-2-[3-((2*R*)-2-{[(2*R*)-2-hydroxy-2-(8-hydroxy-2-oxo-1,2-dihydroquinolin-5-yl)ethyl]amino}propyl)phenyl]acetamide;
*N*-(3,4-dimethylbenzyl)-2-[3-((2*R*)-2-{[(2*R*)-2-hydroxy-2-(8-hydroxy-2-oxo-1,2-dihydroquinolin-5-yl)ethyl]amino}propyl)phenyl]acetamide;
*N*-(2-chloro-6-methylbenzyl)-2-[3-((2*R*)-2-{[(2*R*)-2-hydroxy-2-(8-hydroxy-2-oxo-1,2-dihydroquinolin-5-yl)ethyl]amino}propyl)phenyl]acetamide;
*N*-(3-chloro-4-methylbenzyl)-2-[3-((2*R*)-2-{[(2*R*)-2-hydroxy-2-(8-hydroxy-2-oxo-1,2-dihydroquinolin-5-yl)ethyl]amino}propyl)phenyl]acetamide;
*N*-(3,4-dichlorobenzyl)-2-[3-((2*R*)-2-{[(2*R*)-2-hydroxy-2-(8-hydroxy-2-oxo-1,2-dihydroquinolin-5-yl)ethyl]amino}propyl)phenyl]acetamide;
*N*-[2-fluoro-5-(trifluoromethyl)benzyl]-2-[3-((2*R*)-2-{[(2*R*)-2-hydroxy-2-(8-hydroxy-2-oxo-1,2-dihydroquinolin-5-yl)ethyl]amino}propyl)phenyl]acetamide;
*N*-[2-(4-chlorophenyl)ethyl]-2-[3-((2*R*)-2-{[(2*R*)-2-hydroxy-2-(8-hydroxy-2-oxo-1,2-dihydroquinolin-5-yl)ethyl]amino}propyl)phenyl]acetamide;
2-[3-((2*R*)-2-{[(2*R*)-2-hydroxy-2-(8-hydroxy-2-oxo-1,2-dihydroquinolin-5-yl)ethyl]amino}propyl)phenyl]-*N*-(3-phenylpropyl)acetamide;
*N*-(cyclohexylmethyl)-2-[3-(2-{[(2*R*)-2-hydroxy-2-(8-hydroxy-2-oxo-1,2-dihydroquinolin-5-yl)ethyl]amino}-2-methylpropyl)phenyl]acetamide;
*N*-(cyclohexylmethyl)-*N*-ethyl-2-[3-(2-{[(2*R*)-2-hydroxy-2-(8-hydroxy-2-oxo-1,2-dihydroquinolin-5-yl)ethyl]amino}-2-methylpropyl)phenyl]acetamide;
*N*-(3,4-dimethylbenzyl)-2-[3-(2-{[(2*R*)-2-hydroxy-2-(8-hydroxy-2-oxo-1,2-dihydroquinolin-5-yl)ethyl]amino}-2-methylpropyl)phenyl]acetamide;
*N*-(2-chloro-6-methylbenzyl)-2-[3-(2-{[(2*R*)-2-hydroxy-2-(8-hydroxy-2-oxo-1,2-dihydroquinolin-5-yl)ethyl]amino}-2-methylpropyl)phenyl]acetamide;
*N*-(3,4-dichlorobenzyl)-2-[3-(2-{[(2*R*)-2-hydroxy-2-(8-hydroxy-2-oxo-1,2-dihydroquinolin-5-yl)ethyl]amino}-2-methylpropyl)phenyl]acetamide;
*N*-(3,5-dichlorobenzyl)-2-[3-(2-{[(2*R*)-2-hydroxy-2-(8-hydroxy-2-oxo-1,2-dihydroquinolin-5-yl)ethyl]amino}-2-methylpropyl)phenyl]acetamide;
*N*-[2-fluoro-5-(trifluoromethyl)benzyl]-2-[3-(2-{[(2*R*)-2-hydroxy-2-(8-hydroxy-2-oxo-1,2-dihydroquinolin-5-yl)ethyl]amino}-2-methylpropyl)phenyl]acetamide;
2-[3-(2-{[(2*R*)-2-hydroxy-2-(8-hydroxy-2-oxo-1,2-dihydroquinolin-5-yl)ethyl]amino}-2-methylpropyl)phenyl]-*N*-[2-(methylthio)benzyl]acetamide;
2-[3-(2-{[(2*R*)-2-hydroxy-2-(8-hydroxy-2-oxo-1,2-dihydroquinolin-5-yl)ethyl]amino}-2-methylpropyl)phenyl]-*N*-[4-(methylthio)benzyl]acetamide;
*N*-[2-(4-chlorophenyl)ethyl]-2-[3-(2-{[(2*R*)-2-hydroxy-2-(8-hydroxy-2-oxo-1,2-dihydroquinolin-5-yl)ethyl]amino}-2-methylpropyl)phenyl]acetamide, and,
2-[3-(2-{[(2*R*)-2-hydroxy-2-(8-hydroxy-2-oxo-1,2-dihydroquinolin-5-yl)ethyl]amino}-2-methylpropyl)phenyl]-*N*-(3-phenylpropyl)acetamide.

16. A pharmaceutical composition comprising at least an effective amount of a compound of the formula (1) as described in any one of claims 1 to 15 or a pharmaceutically acceptable salt or derived form thereof.

17. A pharmaceutical composition according to claim 16, further comprising one or more pharmaceutically acceptable excipients and/or additives.

18. A compound of the formula (1) as described in claim 1 or a pharmaceutically acceptable salt, derived form or composition thereof, for use as a medicament.

19. A compound of the formula (1) as described in claim 1 or a pharmaceutically acceptable salt, derived form or composition thereof, for use in the treatment of diseases, disorders, and conditions in which the β2 receptor is involved.

20. A compound of the formula (1) as described in claim 1 or a pharmaceutically acceptable salt, derived form or composition thereof, for use in the treatment of diseases, disorders, and conditions selected from the group consisting of :
• asthma of whatever type, etiology, or pathogenesis, in particular asthma that is a member selected from the group consisting of atopic asthma, non-atopic asthma, allergic asthma, atopic bronchial IgE-mediated asthma, bronchial asthma, essential asthma, true asthma, intrinsic asthma caused by pathophysiologic disturbances, extrinsic asthma caused by environmental factors, essential asthma of unknown or inapparent cause, non-atopic asthma, bronchitic asthma, emphysematous asthma, exercise-induced asthma, allergen induced asthma, cold air induced asthma, occupational asthma, infective asthma caused by bacterial, fungal, protozoal, or viral infection, non-allergic asthma, incipient asthma, wheezy infant syndrome and bronchiolytis,
• chronic or acute bronchoconstriction, chronic bronchitis, small airways obstruction, and emphysema,
• obstructive or inflammatory airways diseases of whatever type, etiology, or pathogenesis, in particular an obstructive or inflammatory airways disease that is a member selected from the group consisting of chronic eosinophilic pneumonia, chronic obstructive pulmonary disease (COPD), COPD that includes chronic bronchitis, pulmonary emphysema or dyspnea associated or not associated with COPD, COPD that is **characterized by** irreversible, progressive airways obstruction, adult respiratory distress syndrome (ARDS), exacerbation of airways hyper-reactivity consequent to other drug therapy and airways disease that is associated with pulmonary hypertension,
• bronchitis of whatever type, etiology, or pathogenesis, in particular bronchitis that is a member selected from the group consisting of acute bronchitis, acute laryngotracheal bronchitis, arachidic bronchitis, catarrhal bronchitis, croupus bronchitis, dry bronchitis, infectious asthmatic bronchitis, productive bronchitis, staphylococcus or streptococcal bronchitis and vesicular bronchitis,
• acute lung injury,
• bronchiectasis of whatever type, etiology, or pathogenesis, in particular bronchiectasis that is a member selected from the group consisting of cylindric bronchiectasis, sacculated bronchiectasis, fusiform bronchiectasis, capillary bronchiectasis, cystic bronchiectasis, dry bronchiectasis and follicular bronchiectasis.

21. The use of a compound of the formula (1) as described in claim 1 or of a pharmaceutically acceptable salt, derived form or composition thereof, for the manufacture of a drug having a β2 agonist activity.

22. The use of a compound of the formula (1) as described in claim 1 or of a pharmaceutically acceptable salt, solvate or composition thereof, for the manufacture of a drug for the treatment of diseases, disorders, and conditions selected from the group as described in claim 20.

23. A method of treatment of a mammal, including a human being, with a β2 agonist including treating said mammal with an effective amount of a compound of the formula (1) as described in claim 1 or with a pharmaceutically acceptable salt, derived form or composition thereof.

24. A method according to claim 22 where the disease, disorder or condition is selected from the group as described in claim 20.

25. Combination of a compound according to any one of claims 1 to 15 with other therapeutic agent(s) selected from:
(a) 5-Lipoxygenase (5-LO) inhibitors or 5-lipoxygenase activating protein (FLAP) antagonists,
(b) Leukotriene antagonists (LTRAs) including antagonists of LTB₄, LTC₄, LTD₄, and LTE₄,
(c) Histamine receptor antagonists including H1 and H3 antagonists,
(d) α₁- and α₂-adrenoceptor agonist vasoconstrictor sympathomimetic agents for decongestant use,
(e) muscarinic M3 receptor antagonists or anticholinergic agents,
(f) PDE inhibitors, e.g. PDE3, PDE4 and PDE5 inhibitors,
(g) Theophylline,
(h) Sodium cromoglycate,
(i) COX inhibitors both non-selective and selective COX-1 or COX-2 inhibitors (NSAIDs),
(j) Oral and inhaled glucocorticosteroids,
(k) Monoclonal antibodies active against endogenous inflammatory entities,
(l) Anti-tumor necrosis factor (anti-TNF-α) agents,
(m)Adhesion molecule inhibitors including VLA-4 antagonists,
(n) Kinin-B₁ - and B₂-receptor antagonists,
(o) Immunosuppressive agents,
(p) Inhibitors of matrix metalloproteases (MMPs),
(q) Tachykinin NK₁, NK₂ and NK₃ receptor antagonists,
(r) Elastase inhibitors,
(s) Adenosine A2a receptor agonists,
(t) Inhibitors of urokinase,
(u) Compounds that act on dopamine receptors, e.g. D2 agonists,
(v) Modulators of the NFκβ pathway, e.g. IKK inhibitors,
(w) modulators of cytokine signalling pathyways such as p38 MAP kinase or syk kinase,
(x) Agents that can be classed as mucolytics or anti-tussive, and
(y) Antibiotics.
